# EUROPEAN PATENT SPECIFICATION

(11) **EP 3 274 052 B1**
(45) Date of publication and mention of the grant of the patent: **14.01.2026**
(21) Application number: 16716931.7
(22) Date of filing: 24.03.2016
(51) Int. Cl.: A61K 35/747, A61K 35/744, A61P 35/00

(54) **HISTAMINE-DAGK-PRODUCING BACTERIAL STRAINS AND THEIR USE IN COLORECTAL CANCER THERAPY**
HISTAMIN-DAGK-PRODUZIERENDE BAKTERIENSTÄMME UND IHRE VERWENDUNG IN DER KOLOREKTALKARZINOMTHERAPIE
SOUCHES BACTÉRIENNES PRODUCTRICES D'HISTAMINE-DAGK ET LEUR UTILISATION DANS LE TRAITEMENT DU CANCER COLORECTAL

(30) Priority: 26.03.2015 US 201562138491 P
(43) Date of publication of application: 31.01.2018
(73) Proprietor: Biogaia AB, 103 64 Stockholm (SE)
(72) Inventor: VERSALOVIC, James, Bellaire, Texas TX 77401 (US); MÖLLSTAM, Bo, SE-443 31 Lerum (SE); GAO, Chunxu, Houston, Texas TX 77054 (US); GANESH, Bhanu, Houston, Texas TX 77054 (US)
(74) Representative: Barker Brettell Sweden AB
(86) International application number: PCT/SE2016/050253
(87) International publication number: WO 2016/153422

(56) References cited:
- WO-A1-2006/110088
- WO-A1-2011/095526
- US-A1- 2013 022 586
- Y. LIU ET AL: "Lactobacillus reuteri strains reduce incidence and severity of experimental necrotizing enterocolitis via modulation of TLR4 and NF- B signaling in the intestine", AMERICAN JOURNAL OF PHYSIOLOGY: GASTROINTESTINAL AND LIVER PHYSIOLOGY., vol. 302, no. 6, 1 March 2012 (2012-03-01), US, pages G608 - G617, XP055281906, ISSN: 0193-1857, DOI: 10.1152/ajpgi.00266.2011
- XIANG DONG YANG ET AL: "Histamine deficiency promotes inflammation-associated carcinogenesis through reduced myeloid maturation and accumulation of CD11b+Ly6G+ immature myeloid cells", NATURE MEDICINE., vol. 17, no. 1, 19 December 2010 (2010-12-19), US, pages 87 - 95, XP055282233, ISSN: 1078-8956, DOI: 10.1038/nm.2278
- CHANDRA IYER ET AL: "Probiotic Lactobacillus reuteri promotes TNF-induced apoptosis in human myeloid leukemia-derived cells by modulation of NF-[kappa]B and MAPK signalling", CELLULAR MICROBIOLOGY, vol. 10, no. 7, 1 July 2008 (2008-07-01), GB, pages 1442 - 1452, XP055281950, ISSN: 1462-5814, DOI: 10.1111/j.1462-5822.2008.01137.x
- KAHOULI I ET AL: "Screening of Lactobacillus reuteri strains for their short chain fatty acids production, stability and potential in colorectal cancer: In-vitro analysis", EUROPEAN JOURNAL OF CANCER, PERGAMON, GB, vol. 50, no. Suppl.5, 1 July 2014 (2014-07-01), pages S212, XP009190601, ISSN: 0959-8049
- ZHENGYU FANG ET AL: "Attenuated expression of HRH4 in colorectal carcinomas: a potential influence on tumor growth and progression", BMC CANCER, BIOMED CENTRAL, LONDON, GB, vol. 11, no. 1, 24 May 2011 (2011-05-24), pages 195, XP021103363, ISSN: 1471-2407, DOI: 10.1186/1471-2407-11-195
- T KUBOTA ET AL: "Cimetidine modulates the antigen presenting capacity of dendritic cells from colorectal cancer patients", BRITISH JOURNAL OF CANCER, 1 January 2002 (2002-01-01), pages 1257 - 1261, XP055282196, Retrieved from the Internet <URL:http://www.nature.com/bjc/journal/v86/n8/pdf/6600233a.pdf> DOI: 10.1038/sj/bjc/6600233

## Description

### TECHNICAL FIELD OF THE INVENTION

The present invention generally relates to lactic acid bacterial strains, and in particular to histamine-producing lactic acid bacterial strain and to their use in, for instance, cancer.

### BACKGROUND OF THE INVENTION

Earlier evidence has been collected indicating that histamine can modulate proliferation of different normal and malignant cells. High histamine biosynthesis and content together with histamine receptors have been reported in different human cancers including melanoma, colon and breast cancer
Colorectal cancer (CRC), also referred to as colon cancer, rectal cancer, or bowel cancer, is the third most common cancer and the third leading cause of cancer related mortality. Colorectal carcinogenesis has been associated with both genetic and environmental factors. Colorectal cancer is cancer in the colon and/or rectum.

*Lactobacillus reuteri* is a commensal intestinal Firmicute and probiotic that is widely prevalent in the gastrointestinal tracts of diverse avian and mammalian species. This organism is considered to be generally recognized as safe (GRAS) and beneficial microbe, and has been used globally as a probiotic for approximately two decades. *L. reuteri* has been reported to suppress pro-inflammatory cytokines in intestinal epithelial cells, monocytes, and intestinal inflammation in different rodent models.

WO 2013/011137 discloses selection of specific probiotic lactic acid bacterial strains producing histamine and the use of such strains for beneficial effects for mammals. The selected bacterial strains may be used in the local production of histamine in mammals, in particular for use in the treatment or prophylaxis of inflammatory conditions.

Iyer et al., Probiotic Lactobacillus reuteri promotes TNF-induced apoptosis in human myeloid leukemia-derived cells by modulation of NF-kappaB and MAPK signalling, Cellular Microbiology 10(7): 1442-1452 (2008) discloses that *L. reuteri* ATCC PTA 6475 secrete factors that potentiate apoptosis in myeloid leukemia-derived cells induced by tumor necrosis factor (TNF). The improved understanding of the *L. reuteri*-mediated effects on apoptotic signalling pathways may facilitate development of future probiotics-based regimens for prevention of colorectal cancer and inflammatory bowel disease.

Liu et al., Lactobacillus reuteri strains reduce incidence and severity of experimental necrotizing enterocolitis via modulation of TLR4 and NF-κB signaling in the intestine, American Journal of Physiology: Gastrointestinal and Liver Physiology 302(6): G608-G617 (2011) demonstrate that *L. reuteri* strains DSM 17938 and ATCC PTA 4659 have potential therapeutic value in a necrotizing enterocolitis (NEC) model and in enteritis associated with cow milk feeding, and could thereby be used in treatment of NEC.

WO 2011/095526 discloses a method of improving immunomodulatory properties of Lactobacillus strains using growth media with a specific primary carbon source, including a method of increasing the anti-inflammatory effect of non-pathogenic anti-inflammatory bacterial strains, by the use of specific growth conditions.

US 2013/0022586 discloses a method of selecting specific probiotic lactic acid bacteria producing histamine and the use of such strains for beneficial effects for mammals. The method includes selecting a lactic acid bacterial strain for use in the local production of histamine in a mammal, and further comprises screening bacteria for the presence of an active histidine operon and selecting a strain which has an active histidine operon and is capable of producing histamine. Also products comprising the strains obtainable by the selection methods of the invention are disclosed for use in the local production of histamine in a mammal, in particular for use in the treatment or prophylaxis of inflammatory conditions.

WO 2006/110088 discloses strains of lactic acid bacteria selected for their capability of reducing inflammation, such as intestinal bowel disease, a method of selecting such strains, and products containing such strains.

Dong Yang et al., Histamine deficiency promotes inflammation-associated carcinogenesis through reduced myeloid maturation and accumulation of CD11b+Ly6G+ immature myeloid cells, Nature Medicine 17(1): 87-95 (2011) discloses that Hdc-knockout mice show a high rate of colon and skin carcinogenesis. Exogenous histamine induced differentiation of immature myeloid cells (IMCs) and suppressed their ability to support the growth of tumor allografts. The data indicated key roles for *Hdc* and histamine in myeloid cell differentiation and CD11b⁺Ly6G⁺ IMCs in early cancer development.

### SUMMARY OF THE INVENTION

The invention herein discloses histamine-producing bacterial strains for use in the treatment of colorectal cancer.

The inventors have found out that histamine-producing probiotic bacteria are capable of reducing the frequency and severity of inflammation-associated CRC in *Hdc*^{*-*/*-*} of colorectal cancer. *L. reuteri* ATCC PTA-6475 significantly decreased the number and size of colorectal or colon tumors. Meanwhile, an isogenic *hdcA* mutant of *L. reuteri* ATCC PTA-6475, which lacks histamine producing activity, did not show such effects, indicating a significant role of the bacterial *hdcA* gene in the gastrointestinal microbiome and production of histamine for suppression of colorectal tumorigenesis.

Accordingly, an aspect of the embodiments relates to a histamine-producing lactic acid bacterial strain for use in prophylaxis, inhibition, treatment or reducing a risk of relapse of colorectal cancer. The histamine-producing lactic acid bacterial strain comprises an active histidine operon and the histamine-producing lactic acid bacterial strain expresses a gene encoding a diacylglycerol kinase (DagK) and produces histamine and produces DagK.

Another aspect of the embodiments relates a histamine-producing lactic acid bacterial strain for use as adjuvant in a cancer treatment of colorectal cancer. The cancer treatment is selected from a group consisting of radiotherapy and chemotherapy. The histamine-producing lactic acid bacterial strain comprises an active histidine operon and the histamine-producing lactic acid bacterial strain expresses a gene encoding a DagK and produces histamine and produces DagK.

Another aspect of the embodiments relates to a method of selecting a lactic acid bacterial strain for use in prophylaxis, inhibition, treatment or reducing a risk of relapse of colorectal cancer. The method comprises screening lactic acid bacteria for presence of an active histidine operon and presence of expression of a gene encoding a DagK. The method also comprises selecting a lactic acid bacterial strain for use in prophylaxis, inhibition, treatment or reducing a risk of relapse of colorectal cancer identified as a lactic acid bacterial strain having an active histidine operon and producing histamine and producing DagK.

Further aspects of the embodiments relates to a new *L. reuteri* strain *Lactobacillus reuteri* DSM 32273, such a *L. reuteri* DSM 32273 strain for use as a medicament and in particular for use in prophylaxis, inhibition, treatment or reducing a risk of relapse of colorectal cancer.

### DESCRIPTION OF THE DRAWINGS

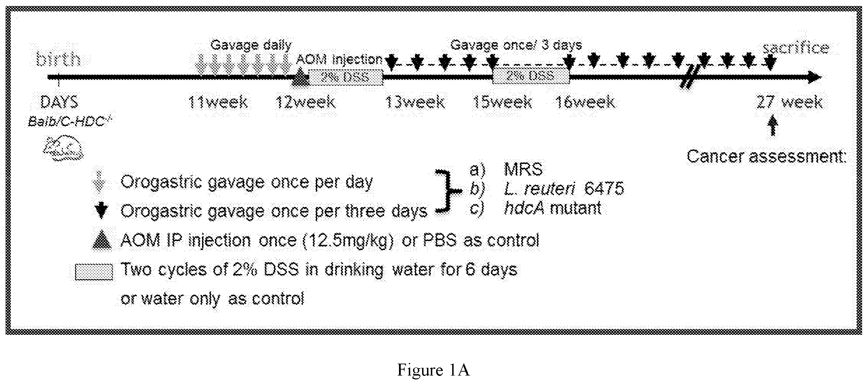

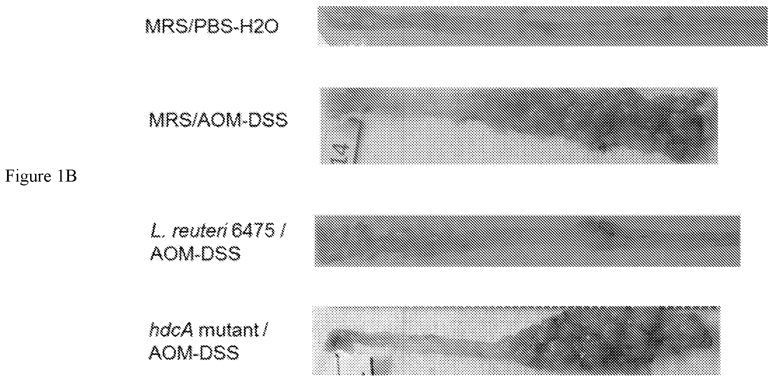

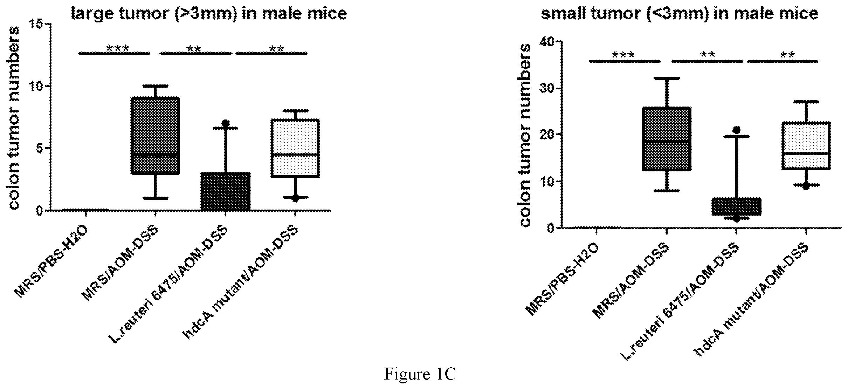

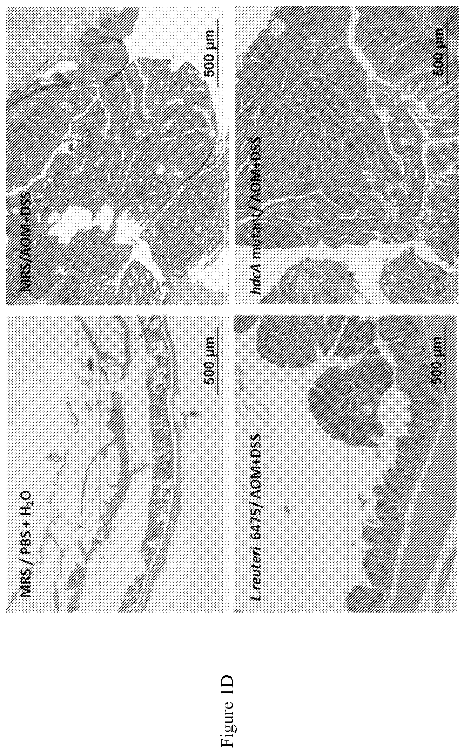

**Figure 1****. Wild *type L. reuteri* ATCC PTA-6475, but not its *hdcA* Mutant, Attenuates AOM/DSS Induced Colon Cancer *in vivo.*** (A) Time line of the mouse experiments. Eleven week old *Hdc*^{*-*/*-*} BALB/c mice were randomly divided into four groups, including a negative control group, a positive control group, a *L. reuteri* ATCC PTA-6475-treated group and a *L. reuteri hdcA* mutant-treated group. The mice in the *L. reuteri* ATCC PTA-6475-treated group or *hdcA* mutant-treated group received 5×10⁹ CFU wild type *L. reuteri* ATCC PTA-6475 or the isogenic *L. reuteri hdcA* mutant strain, respectively, in 0.2 ml MRS by orogastric gavage once per day for seven days and once per three days afterwards. The mice in the negative and positive control groups received rich microbiologic media (MRS) only. At twelve weeks of age, these mice were challenged with one dose of AOM (12.5 mg/kg) by intraperitoneal injection followed by two cycles of 2% DSS treatment in drinking water for 6 days with two-week recovery periods of drinking water only between DSS administration dates. The mice in the negative control group received one dose of PBS and drinking water only, instead of AOM and DSS. The mice were sacrificed at 27-weeks of age and colonic carcinogenesis was evaluated in each group. (B) Representative colon images of mice in the negative control group (MRS/PBS-H2O), positive control group (MRS/AOM-DSS), *L. reuteri* ATCC PTA-6475-treated group *(L. reuteri* 6475/AOM-DSS) and isogenic *L. reuteri hdcA* mutant-treated group (*hdcA* mutant/AOM-DSS). (C) Numbers of both large (>3 mm) and small (<3 mm) colonic tumors from mice in each group mentioned above. Data is presented as box and whiskers plots showing the median and 10th and 90th percentiles (***P<0.01,* ****p<0.001,* n=8~10 for each group). (D) Representative microscopic colon images (H&E stained) from mice in the negative control group (MRS/PBS-H₂O), positive control group (MRS/AOM-DSS), wild type *L. reuteri* ATCC PTA-6475-treated group *(L. reuteri* 6475/AOM-DSS) and the isogenic *L. reuteri hdcA* mutant-treated group (*hdcA* mutant/AOM-DSS).

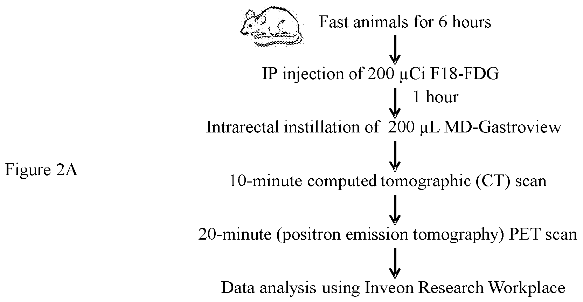

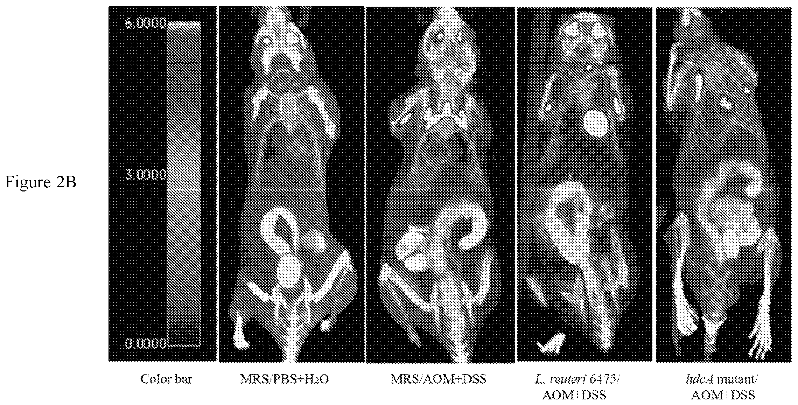

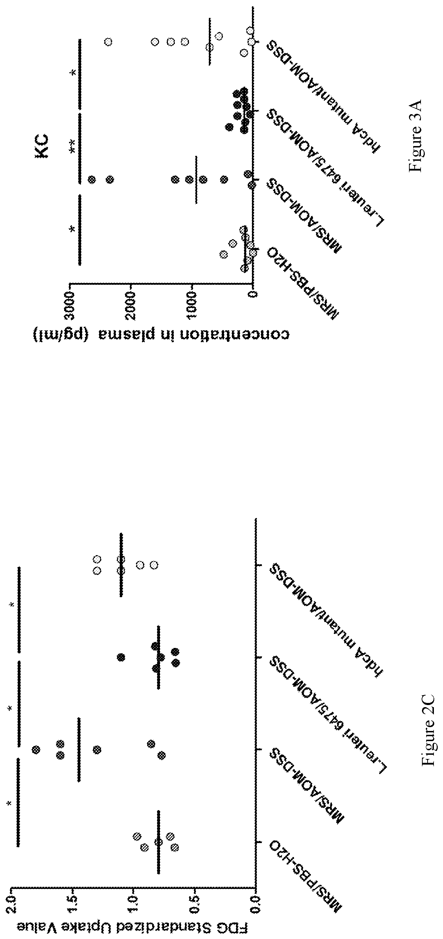

**Figure 2****. Detection of Colonic Tumorigenesis Attenuation by PET Imaging.** (A) PET imaging procedures. (B) Representative mouse images captured by PET/CT scanning in each group. The code bar represents FDG signal intensity. (C) Quantification of FDG signals in the whole mouse colon using SUV in each group showed that *L. reuteri* ATCC PTA-6475 administration significantly reduced FDG intensities in the mouse colon, compared to MRS media control but the isogenic *L. reuteri hdcA* mutant lacked such effects (analyzed blindly). Data is presented as scatter plots (**P* < *0.05,* n=6 for each group).

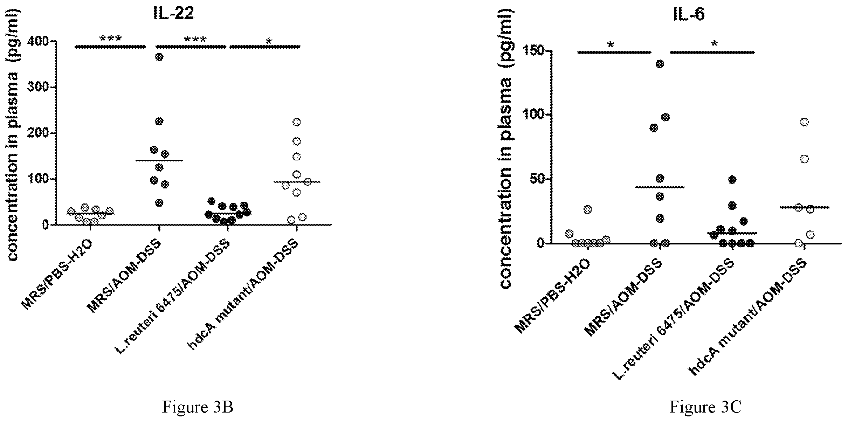

**Figure 3****. *L. reuteri* ATCC PTA-6475 Administration Affects Cytokine Production in Murine Plasma.** *L. reuteri* ATCC PTA-6475 administration significantly decreased the pro-inflammatory cytokine KC (A), IL-22 (B) and IL-6 (C) production in male *Hdc*^{*-*/*-*} mouse plasma in protein level determined by Luminex assay, whereas the isogenic *L. reuteri hdcA* mutant, which lacks the histamine-producing capacity, did not show such effects. Data is presented as scatter plots (*P < *0.05, **P<0.01, ***p<0.001,* n=8~10 for each group).

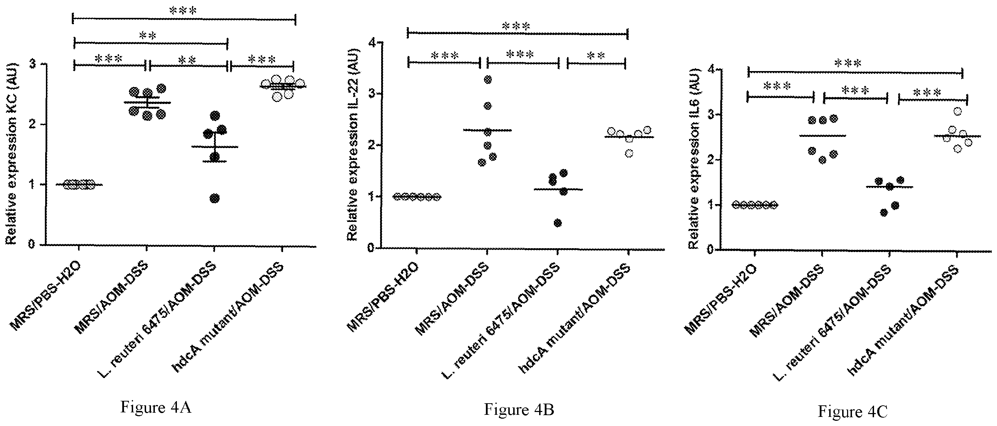

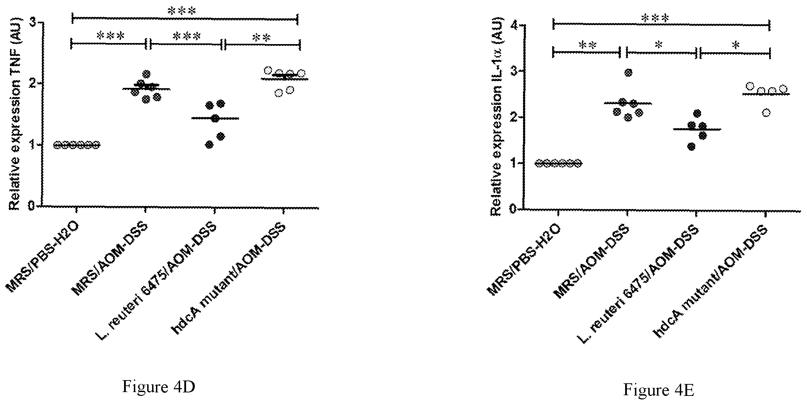

**Figure 4****. *L. reuteri* ATCC PTA-6475 Administration Affects Cytokine Gene Expression in the Colon.** *L. reuteri* ATCC PTA-6475 administration significantly decreased the pro-inflammatory cytokine KC (A), IL-22 (B), IL-6 (C), TNF (D), and IL-1α (E) gene expression in male *Hdc*^{*-*/*-*} mouse colonic mucosa in mRNA level determined by RT-qPCR, whereas the isogenic *L. reuteri hdcA* mutant, which lacks the histamine-producing capacity, did not show such effects. Data is presented as scatter plots (**P* < *0.05, **P<0.01, ***p<0.001,* n=5~6 for each group).

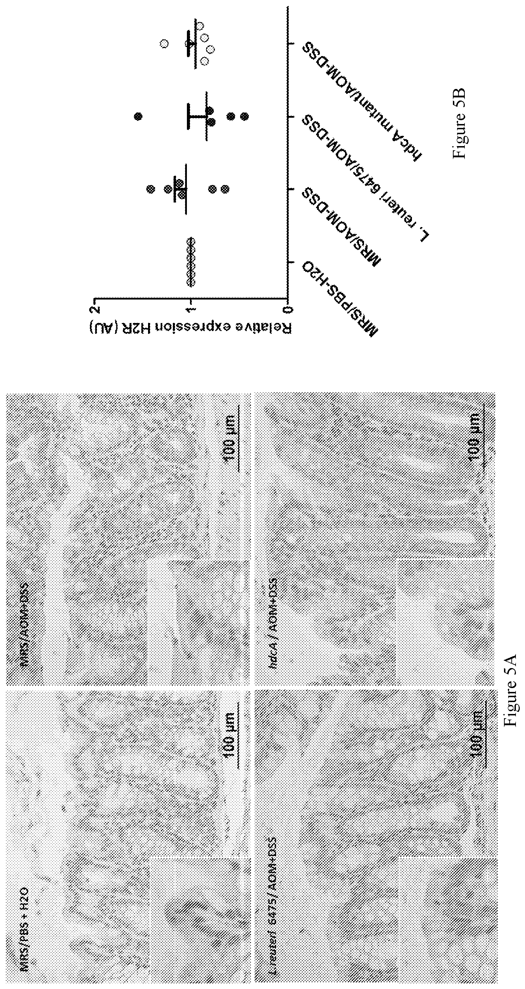

**Figure 5****. H2R Expression Was Reduced by AOM/DSS Challenge and Induced by *L*. *reuteri.*** (A) Immunohistochemistry studies using H2R specific antibody showed that H2R was expressed in the colon of *Hdc*^{*-*/*-*} mice. AOM and DSS treatment reduced the intensity of H2R compared to healthy controls and *L. reuteri* administration induced H2R expression. (B) H2R gene expression in colonic mucosa was not significantly changed by AOM/DSS challenge or *L. reuteri* administration in *Hdc*^{*-*/*-*} male mice (n=5~6 for each group).

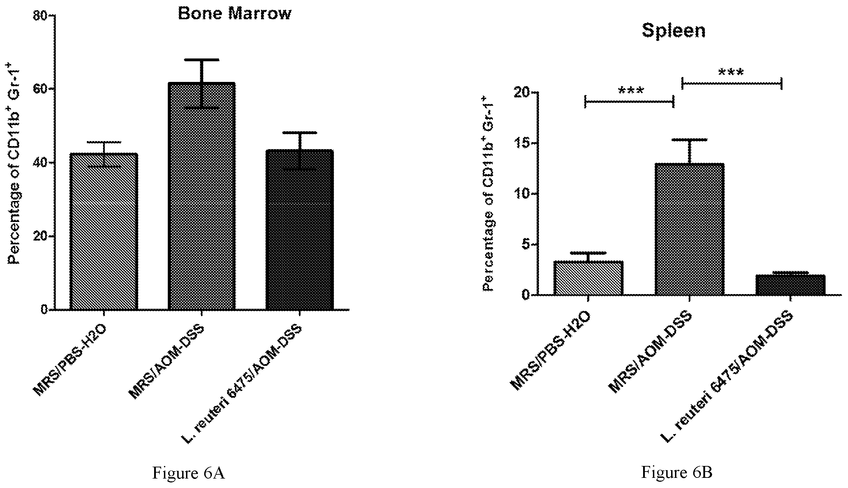

**Figure 6****. CD11b⁺Gr-1⁺ IMCs in the Spleen Were Reduced by *L. reuteri* ATCC PTA-6475 Administration.** Flow cytometric analysis acquired from the bone marrow (A) and spleen (B) samples of *Hdc*^{*-*/*-*} male mice showed that AOM/DSS treatment significantly increased the percentage of CD11b⁺Gr-1⁺ IMCs compared to healthy controls in the spleen. *L. reuteri* ATCC PTA-6475 administration in AOM/DSS challenged mice significantly decreased the percentage of CD11b⁺Gr-1⁺ IMCs in the spleen, compared to the mice that did not receive bacteria *(***p<0.001,* means ± s.d.; n=3~4 for each group).

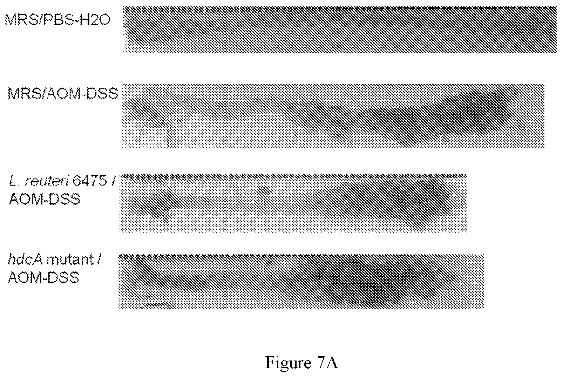

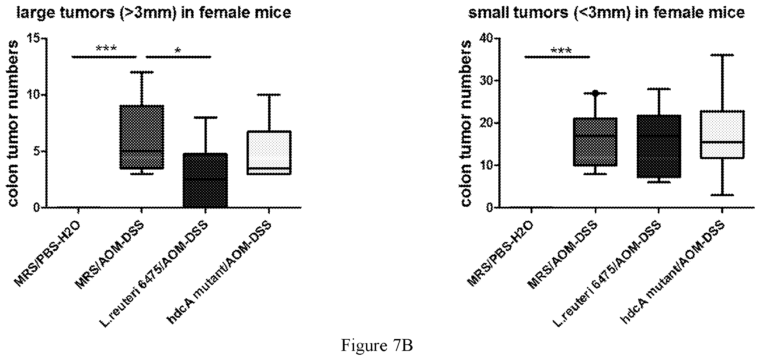

**Figure 7****. *L. reuteri* ATCC PTA-6475 Reduced Large Colonic Tumors in *Hdc*^{*-*/*-*} Female Mice.** (A) Representative colon images from mice in negative control group (MRS/PBS-H2O), positive control group (MRS/AOM-DSS), *L. reuteri* ATCC PTA-6475-treated group *(L. reuteri* 6475/AOM-DSS) and *hdcA* mutant-treated group (*hdcA* mutant/AOM-DSS). (B) The number of large (>3 mm) colon tumors from *Hdc*^{*-*/*-*} female mice were significantly decreased by *L. reuteri* ATCC PTA-6475 administration, but the number of small (<3 mm) colon tumors from *Hdc*^{*-*/*-*}female mice were not significantly changed. Data is presented as box and whiskers showing the median and 10 and 90 percentiles *(*P<0.05, ***p<0.001,* n=8~10 for each group).

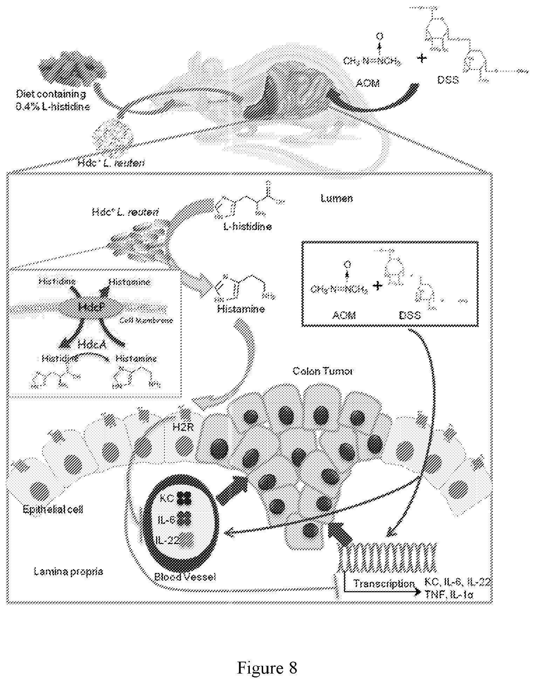

**Figure 8****. Mechanism of *L. reuteri* in Model of Inflammation-Associated Carcinogenesis.** The figure schematically illustrates a potential mechanism of the probiosis of *L. reuteri* ATCC PTA-6475 in the mouse model of inflammation-associated carcinogenesis.

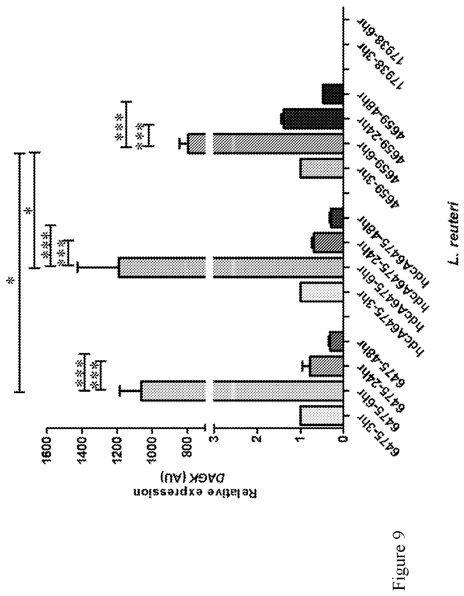

**Figure 9****. *L. reuteri* WT and *hdcA* Mutant ATCC PTA-6475 and ATCC PTA-4659 Produce Diacylglycerol Kinase (DagK).** Relative mRNA target gene expression levels normalized to the house keeping gene *rpoB* are presented from 3, 6, 24 and 48 hours culture of each bacterium. mRNA obtained from 3 hours culture of each bacterium were set to 1.0 and used as calibrator to identify the relative mRNA fold difference.

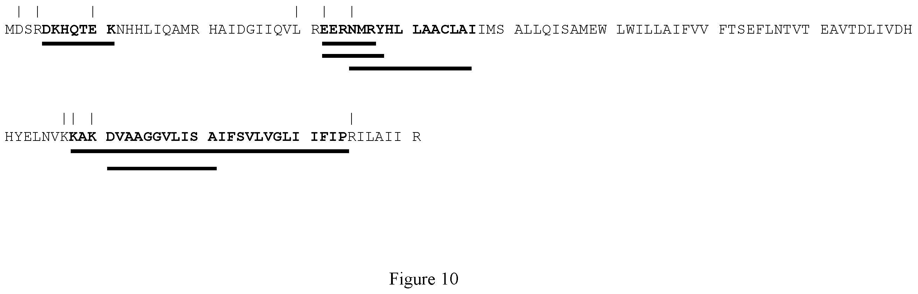

**Figure 10****. *L. reuteri* DagK Amino Acid Sequence.** The amino acid sequence of *L. reuteri* DagK (SEQ ID NO: 25) is shown together with trypsin cleavage sites (vertical lines). The bold amino acids indicate peptide sequences obtained following such trypsin treatment. The black bars indicate peptide sequences found in LC-MS/MS experiments.

### DETAILED DESCRIPTION OF THE INVENTION AND PREFERRED EMBODIMENTS THEREOF

The present invention discloses probiotic histamine-producing bacterial strains for use in colorectal cancer. The present invention discloses selection of probiotic histamine-producing bacterial strains for use in colorectal cancer. The selected histamine producing bacterial strains may be used for local delivery of histamine, as histamine receptor agonists.

An example of the disclosure is to select certain probiotic bacteria capable of producing histamine. These selected bacteria can be used in treatment of cancer, especially histamine dependent cancer, such as for example colorectal cancer.

An embodiment of the present invention is to select certain probiotic bacteria capable of producing histamine and also capable of producing diacylglycerl kinase (DagK), such as producing and secreting DagK, or at least release DagK to have an extracellular effect. These selected bacteria can be used in prophylaxis, inhibition, treatment or reducing a risk of relapse of colorectal cancer.

Another example of the disclosure is to select certain probiotic bacteria capable of producing histamine and capable of producing DagK. These selected bacteria can be used in prophylaxis, inhibition or treatment of an inflammatory condition, such as for example colitis, inflammatory bowel disease (IBD), irritable bowel syndrome (IBS), diverticulosis, gingivitis, mastitis or vaginitis.

In another embodiment of the invention the selected bacterial strains are preferably used for male cancer patients and in yet another embodiment of the invention the bacterial strains are used for male patients with large colon or rectal tumors.

The selected bacterial strains may be used as an adjuvant to classical radiotherapy or chemotherapy.

The selected bacterial strains may also be used as a product to minimize diarrhea and other gastrointestinal disturbances associated with radiotherapy or chemotherapy of cancer and at the same time work as an adjuvant to improve such treatment to reduce the cancer.

Microbe-derived histamine may yield different effects in the host, depending on the activation of specific histamine receptors that differ in their tissue expression patterns. Particularly, regarding CRC, significantly increased histidine decarboxylase (HDC) activity was found in specimens from extirpated human tumors in a series of ten surgical patients with colorectal carcinoma, indicating a significant role of the enzyme activity of HDC in the development of colorectal tumor cells (Garcia-Caballero et al., 1988). On the other hand, the deficiency of HDC was shown to promote inflammation-associated CRC by accumulation of CD11b⁺Gr-1⁺ IMCs (Yang et al., 2011). Regarding H2R, inhibition of H2R through its antagonist increased the survival of patients with CRC (Adams and Morris, 1994; Kelly et al., 1999).

The inventors have found out that histamine-producing bacteria, such as *L. reuteri* ATCC PTA-6475 protected male *Hdc*^{*-*/*-*} mice in an azoxymethane/dextran sodium sulfate (AOM/DSS)-induced inflammation associated colorectal cancer model, as indicated by decreased numbers and sizes of colon tumors assessed by macroscopic and microscopic evaluation of colon, as well as ¹⁸F-FDG live animal PET imaging. Meanwhile, we found that the enzymatic machinery, histidine decarboxylase, must be present in the intestinal microbiome in order to generate histamine as the bioactive compound as indicated by the loss of anti-tumorigenic effects of *hdcA* mutant. Cytokine studies (protein quantities in plasma and mRNA quantities in colonic mucosa) showed a reduction of the inflammation/tumor-associated cytokines by administration of the histamine-producing strain, but not the non-histamine-producing strain, consistent with the phenotypic observations.

We also observed a potential change in H2R expression in the colon of *Hdc*^{*-*/*-*} mice. AOM and DSS treatment reduced the intensity of H2R compared to healthy controls and *L. reuteri* administration induced H2R expression. This observation suggested an association between H2R expression and inflammation or CRC. When mice were treated with AOM/DSS for colon cancer induction, H2R expression is reduced. When *L. reuteri* is administered, H2R expression was induced. Considering that *hdcA* mutant administration also showed an increased H2R expression (not as high as *L. reuteri* ATCC PTA-6475 group) compared to positive control mice that received AOM/DSS but no bacteria, *L. reuteri* might produce some substances in addition to histamine that may induce H2R.

An object of the present invention is histamine and DagK producing bacteria to treat and prevent colorectal (colon) cancer.

Another object is to use certain bacteria for local delivery of histamine as a histamine receptor agonist, possibly delivered together with a specific source of histidine.

In another preferred embodiment the selected bacterial strains are used especially for male patients suffering from colorectal cancer.

In yet another embodiment the selected bacterial strains are used especially for male patients with lager colon or rectal tumors.

In another embodiment the histamine-producing strains are used as an adjuvant to classical radiotherapy or chemotherapy

Herein is described a product comprising the histamine producing strains to minimize diarrhea and other gastrointestinal disturbances associated with radiotherapy or chemotherapy of cancer and at the same time work as an adjuvant to improve such treatment to reduce the histamine dependent cancer

Another feature of AOM/DSS induced colorectal cancer in *Hdc*^{*-*/*-*} mice is an accumulation of CD11b⁺Gr-1⁺ IMCs in the spleen. CD11b⁺Gr-1⁺ IMCs are seen to be accumulated largely in the spleen of cancer-bearing mice (Watanabe et al., 2008) with deficient *Hdc* gene expression (Yang et al., 2011). The inventors have found out that administration of histamine-producing bacteria reduced the CD11b⁺Gr-1⁺ IMCs, which confirmed that exogenous histamine regulated the differentiation of CD11b⁺Gr-1⁺ IMCs.

Based on these investigations, we summarized the potential mechanism of the probiosis of *L. reuteri* ATCC PTA-6475 in the mouse model of inflammation-associated carcinogenesis (Figure 8). Intraperitoneal injection of AOM followed by DSS treatment in drinking water induced inflammation-associated colon cancer as indicated by macroscopically visible colonic tumors, increased quantities of colonic KC, IL-22, IL-6, TNF, and IL-1α by gene expression data, and increased quantities of plasma IL-6, IL-22 and KC. When mice were fed with histidine-containing diet, *hdc⁺ L. reuteri* administered to mice by orogastric gavage converted L-histidine to histamine by histidine decarboxylase (HdcA) and exported histamine to the lumen by histidine/histamine antiporter (HdcP) (Thomas et al., 2012). *L. reuteri* derived histamine activated histamine H2 receptor (H2R) on epithelial cells and triggered the anti-tumorigenic pathways as indicated by suppression KC, IL-22, IL-6, TNF, and IL-1α gene expression in the colon, and IL-6, IL-22 and KC production in plasma. On the other hand, *L. reuteri* ATCC PTA-6475 administration reduced the relative abundance of CD11b⁺Gr-1⁺ IMCs in the spleen, and the reduction of CD11b⁺Gr-1⁺ IMCs also contributed to a potential anti-tumorigenic effect.

### L. reuteri ATCC PTA-6475 but not the hdcA Mutant Attenuates Colonic Carcinogenesis in vivo

To examine whether *L. reuteri ATCC PTA-6475's* colonization has an anti-tumorigenic role, we used AOM plus DSS treatment to induce colitis-associated colon cancer in twelve-week *Hdc*^{*-*/*-*} BALB/c mice (Figure 1A). The severity of colonic tumorigenesis was evaluated 15 weeks after AOM injection by the number and size of colonic tumors. For the males, negative control mice that received buffered saline solution (PBS) and drinking water, instead of AOM and 2% DSS, did not develop tumors. Positive control mice that were challenged with AOM/DSS and gavaged with MRS media, but did not receive exogenous bacteria, developed colonic tumors. Administration of *L. reuteri* ATCC PTA-6475 in its exponential phase significantly reduced the number and size of colonic tumors, compared to the positive control group. However, *hdcA* mutant administration did not show such effects (Figure 1B~1C). For the females, a similar pattern was observed. Negative control mice did not yield colonic tumors, and positive control mice developed colonic tumors. *L. reuteri* ATCC PTA-6475 administration significantly reduced the number of large (>3 mm) colonic tumors compared to the positive control group, but the number of small (<3 mm) tumors was not reduced. Administration of a *L. reuteri hdcA* mutant strain did not reduce the amounts of large or small colorectal tumors (Figure 7).

Histologic analysis of the male colons by H&E staining confirmed the anti-tumorigenic effect of wild-type, histamine generating *L. reuteri* ATCC PTA-6475. Negative control mice showed the expected colonic histology, while positive control mice showed evidence of extensive colon tumors. *L. reuteri* ATCC PTA-6475 treated mice yielded reduced sizes and numbers of colonic tumors, compared to the positive control group, and the isogenic *L. reuteri hdcA* mutant strain did not yield similar effects (Figure 1D).

### PET Imaging Supported the Anti-tumorigenic Effects of L. reuteri ATCC PTA-6475

To further analyze the possible anti-tumorigenic effects of *L. reuteri* in the AOM/DSS-induced mouse model of colon cancer, PET imaging, one of the most powerful noninvasive diagnostic tool for tracing organ functioning, was applied to the mice before sacrificing them (Figure 2A). [¹⁸F]FDG was used as the tracer and its concentration within the body reflects the distribution of glucose uptake and phosphorylation (Brewer et al., 2008). During colonic tumorigenesis, FDG uptake by activated lymphocytes in the colon can be detected by high intensity of tracer signal. In negative control mice, FDG signal was mostly detected in the mouse bladder and upper chest, areas that were proposed to be "normal" body sites that high glucose uptake and metabolism occur (Galitovskiy et al., 2013). Trace amounts of FDG signal were evident in the colon region, indicating low glucose uptake in the colon in healthy mice (Figure 2B~2C). In positive control mice, several hot spots in the colon were observed and the FDG intensity in the whole mouse colon was significantly increased compared to the negative control group (Figure 2B~2C), indicating the detection of colonic tumors and increased glucose uptake in the colons of mice that received AOM plus DSS treatment and gavaged with MRS media. *L*. *reuteri* ATCC PTA-6475-treated mice showed reduced number of hot spots in the colon and significantly decreased FDG intensities compared to positive controls, demonstrate the anti-tumorigenic effect of *L. reuteri* ATCC PTA-6475. Meanwhile, the isogenic *L. reuteri hdcA* mutant did not yield similar effects and showed increased numbers of hot spots in the colon in addition to significantly increased FDG intensities, compared to *L. reuteri* ATCC PTA-6475-treated mice. These results indicate that the lack of an intact bacterial histidine decarboxylase gene in the intestinal microbiome results in the loss of anti-tumorigenic effects, consistent with observed colon numbers and sizes.

### Systemic Cytokine Concentrations in Mouse Plasma were Associated with the Anti-tumorigenic Effects of L. reuteri

Specific pro-inflammatory cytokines have been reported to contribute to the development of colonic tumorogenesis by promoting the formation of a tumor-supportive microenvironment (Landskron et al., 2014). Taking advantage of a Luminex system (Millipore, Billerica, MA, USA), we were able to multiplex (simultaneously measure) analytes in a single microplate using small sample volumes (25 µl). Protein levels of sixteen cytokines (Table 1) in the plasma were measured using four cytokine multiplex kits. Interestingly, we found that three cytokines including KC, IL-22 and IL-6 showed a similar pattern (Figure 3): AOM/DSS treatment in male *Hdc*^{*-*/*-*} mice significantly increased production of these cytokines in plasma compared to the control mice that received PBS/H2O; *L. reuteri* ATCC PTA-6475 administration significantly decreased production of these cytokines, whereas the isogenic *L. reuteri hdcA* mutant, which lacks the histamine-producing capacity, did not decrease these cytokines in AOM/DSS treated *Hdc*^{*-*/*-*} male mice.

KC shares many functional properties with IL-8 (Oquendo et al., 1989), which has been reported to promote colon cancer growth, progression and metastasis (Lee et al., 2012). IL-22 was also shown to promote gastric cancer cell invasion (Fukui et al., 2014; Ji et al., 2014) and colon cancer stemness (Kryczek et al., 2014). IL-6 has been considered as a key regulator of colorectal cancer development (Waldner et al., 2012) and high levels of plasma IL-6 are correlated with a poor prognosis in a variety of cancers including colon cancer (Nagasaki et al., 2014). Based on these reported evidences, the changes of these cytokines in the plasma of different mouse groups in our study are associated and consistent with the disease phenotype: increase of the cytokines associated with CRC induction by AOM/DSS challenge compared to the healthy controls, decrease of the cytokines associated with the attenuation of CRC by histamine-producing *L. reuteri* ATCC PTA-6475 administration, and increase of the cytokines associated with the loss of the anti-tumorigenic effects by non-histamine-producing *hdcA* mutant.

### Cytokine Gene Expression in the Colonic Mucosa was Regulated by L. reuteri Administration

To further investigate the associations between cytokines and CRC severity, we analyzed the gene expression of selected cytokines (Table 2) in colonic mucosal samples by RT-qPCR using GAPDH as the internal standard, in addition to measurement of systemic cytokine quantities in plasma. AOM/DSS challenge significantly induced the gene expression of pro-inflammatory cytokines KC, IL-22, IL-6, TNF, and IL-1α compared to healthy male *Hdc*^{*-*/*-*} mice (Figure 4). *L. reuteri* ATCC PTA-6475 treatment of AOM/DSS challenged mice significantly reduced the relative gene expression of these cytokines whereas the isogenic *L. reuteri hdcA* mutant lacking histamine production yielded increased relative gene expression of these pro-inflammatory cytokines compared to the wild type bacteria gavaged mice. Detection of other cytokine mRNA by qPCR yielded either undetectable results (IL-17) or no significant differences among the groups (IL-12, IL-23 and IFN-γ).

### H2R Expression was Induced by L. reuteri Administration

Histamine-producing *L. reuteri* ATCC PTA-6475 administration attenuated AOM/DSS induced CRC in male *Hdc*^{*-*/*-*} mice whereas non-histamine-producing *hdcA* mutant lost such effects, indicating an important role of luminal histamine in attenuation of CRC. However, the signaling pathway by which histamine may exert its anti-inflammatory and anti-carcinogenic effects is not clear. Histamine is a biogenic amine that exerts various pathophysiological functions via four histamine receptors (H1R, H2R, H3R and H4R) (O'Mahony et al., 2011), and H2R activation has been associated with anti-inflammatory effects (Frei et al., 2013; Jutel et al., 2001; O'Mahony et al., 2011). Moreover, our previous studies showed that *L. reuteri* ATCC PTA-6475 attenuates TNBS-induced colitis by activation of H2R. So in our current study, we investigated the relative H2R expression in different groups of male *Hdc*^{*-*/*-*} mice by immunohistochemistry using H2R specific antibodies. H2R expression was detected in the colons of healthy male *Hdc*^{*-*/*-*} mice, with relatively high intensities in the crypts (Figure 5A). Interestingly, AOM/DSS challenge reduced the relative intensities of H2R in the colon, compared to healthy controls. When the mice that were challenged with AOM/DSS received either *L. reuteri* ATCC PTA-6475 or the isogenic *L. reuteri hdcA* mutant, increased H2R expression was observed compared to the control group that did not receive any bacteria. *L*. *reuteri* ATCC PTA-6475 administration yielded the highest H2R intensity, suggesting that H2R activation may be induced by specific gut microbes and that histamine may play an important role in attenuation of CRC. Reduction of H2R is associated with the development of CRC in AOM/DSS challenged mice and induction of H2R by *L. reuteri* ATCC PTA-6475 administration is associated with attenuated CRC. Since the *hdcA* mutant administration also increased H2R intensities (though not as high as wild type strain), it seems that *L. reuteri* administered to mice may produce a signal, in addition to histamine, that may induce H2R expression in mouse colon.

However, when the H2R expression in mRNA level was investigated by RT-qPCR in colonic mucosa, no significant differences among the groups were observed (Figure 5B). This observation indicates that different quantities of cell surface H2R are due to post-transcriptional differences in protein production and localization.

### L. reuteri ATCC PTA-6475 Downregulated CD11b⁺Gr-1⁺ IMCs in the Spleen

The absence of endogenous histamine leads to increased CD11b⁺Gr-1⁺ IMCs and this is associated with cancer progression in mammals (Yang et al., 2011). In order to assess whether histamine-producing *L. reuteri* ATCC PTA-6475 administration affects the differentiation of IMCs, flow cytometric analysis was performed on the cells derived from bone marrow and spleen specimens samples collected immediately after the male *Hdc*^{*-*/*-*} mice were sacrificed. The percentage of CD11b⁺Gr-1⁺ IMCs in the spleen was significantly increased in AOM/DSS challenged mice compared to the healthy controls (Figure 6). *L. reuteri* ATCC PTA-6475 administration in AOM/DSS challenged mice significantly decreased the percentage of CD11b⁺Gr-1⁺ IMCs compared to the mice that received media only (MRS) controls. These observations are consistent with the phenotypic results that *L. reuteri* ATCC PTA-6475 attenuated AOM/DSS induced CRC. Showing that histamine-producing *L. reuteri* ATCC PTA-6475 administration affects the differentiation of IMCs.

### L. reuteri ATCC PTA-6475, ATCC PTA-4659 and DSM 32273 Express dagK Gene

Wild-type *L. reuteri* ATCC PTA-6475 and the *hdcA* mutant together with *L. reuteri* DSM 32273 and *L. reuteri ATCC PTA-4659* were capable of expressing the *dagK* gene. The *dagK* gene was expressed very high during the elongation phase of the bacteria. Other bacterial strains, including other *L. reuteri* strains, that express the *dagK* gene can be detected using the methods as described herein. This *dagK* gene is lacking in a *L. reuteri* strain DSM 17938 that also cannot produce histamine.

### L. reuteri ATCC PTA-6475 Secrete/Release DagK

The protein DagK expressed by the *dagK* gene in *L. reuteri* ATCC PTA-6475 was found in the supernatant of the culture medium following removal of intact bacterial cells. Accordingly, *L*. *reuteri* ATCC PTA-6475 is capable of producing and secreting, or in other ways releasing DagK to thereby achieve an extracellular DagK effect.

DagK is an enzyme that catalyzes the conversion of diacylglycerol (DAG) to phosphatidic acid (PA) utilizing adenosine triphosphate (ATP) as a source of the phosphate. In non-stimulated cells, DagK activity is low allowing DAG to be used for glycerophospholipid biosynthesis. However, on receptor activation of the phosphoinositide pathway, DagK activity increases driving the conversion of DAG to PA. Conversion of DAG to PA depletes DAG, which otherwise may activate protein kinase C (PKC).

H1R downstream signaling is interrupted by DagK synthesis in *L. reuteri* by inhibiting lipid DAG involved in the signaling. Accordingly, histamine- and DagK-producing lactic acid bacterial strains as disclosed herein suppress pro-inflammatory effects of histamine. This in turn allows only H2R activation by the histamine produced by the bacterial strains. Such H2R activation promotes anti-inflammatory symptoms.

Thus, a lactic acid bacterial strain capable of producing both histamine and DagK causes a suppression of H1R downstream signaling but induces H2R activation. This in turn suppresses the pro-inflammatory effects of histamine and promotes anti-inflammatory symptoms. Bacterial strains with active *dagK* gene expression can produce and optionally secrete, or in other ways release, DagK.

An aspect of the embodiments relates to a histamine-producing lactic acid bacterial strain for use in prophylaxis, inhibition, treatment or reducing a risk of relapse of colorectal cancer. The histamine-producing lactic acid bacterial strain comprises an active histidine operon and the histamine-producing lactic acid bacterial strain expresses a gene encoding a DagK and produces histamine and produces DagK.

The histamine-producing lactic bacterial strains of the embodiments as disclosed herein have beneficial characteristics that can be exploited in connection in colorectal cancer patients, or patients having a risk of developing colorectal cancer. This means that the histamine-producing lactic acid bacterial strains of the embodiments can be used to treat colorectal cancer in a patient.

The treatment with histamine-producing lactic acid bacterial strains could be combined with other cancer treatments including, but not limited to, radiotherapy, chemotherapy and surgery. The histamine-producing lactic acid bacterial strains may in such a case be used as an adjuvant in a cancer treatment. Accordingly, another aspect of the embodiments relates to a histamine-producing lactic acid bacterial strain for use as adjuvant in a cancer treatment of colorectal cancer. The cancer treatment is selected from a group consisting of radiotherapy and chemotherapy. The histamine-producing lactic acid bacterial strain comprises an active histidine operon and the histamine-producing lactic acid bacterial strain expresses a gene encoding a DagK and produces histamine and produces DagK.

The histamine-producing lactic acid bacterial strains of the embodiments also have, in addition to the cancer treatment properties *per se,* beneficial properties to patients undergoing or subject to cancer treatment, in particular patients subject to radiotherapy and/or chemotherapy. In particular, the histamine-producing lactic acid bacterial strains are useful to combat or treat gastrointestinal disturbances associated with, such as caused by, the cancer treatment.

In an example of the disclosure, the histamine-producing lactic acid bacterial strain is for use in prophylaxis, inhibition or treatment of diarrhea associated with the cancer treatment. In a particular embodiment, the cancer treatment is a cancer treatment to treat colorectal cancer, i.e. the patient subject to the cancer treatment is a patient suffering from colorectal cancer.

The administration of the histamine-producing lactic acid bacterial strains to the patient does, however, not necessarily have to cause a 100 % treatment of the colorectal cancer in the patient, i.e. resulting in a patient without any detectable tumors at all. Thus, the histamine-producing lactic acid bacterial strains could be used to inhibit or reduce colorectal cancer in the patient. For instance, experimental data as presented herein indicate that the histamine-producing lactic acid bacterial strains are capable of decreasing the numbers of tumors and decreasing the sizes of the tumors. Accordingly, inhibition or reduction of colorectal cancer comprises, in embodiments, decreasing the numbers of tumors in the patient, decreasing the sizes of tumors in the patient or decreasing the numbers and sizes of tumors in the patient. Thus, in an embodiment, the histamine-producing lactic acid bacterial strain is for use in reducing numbers of and/or sizes of tumors in a patient suffering from colorectal cancer.

The histamine-producing lactic acid bacterial strains of the embodiments could also, or alternatively, be used in prophylaxis, i.e. to reduce the risk of a patient developing colorectal cancer. The patient could, for instance, be a patient having a predisposition to colorectal cancer, such as a genetic or heredity predisposition to colorectal cancer. The histamine-producing lactic acid bacterial strains could then be administered to such a patient to prevent or at least reduce the risk of the patient suffering from colorectal cancer.

The histamine-producing lactic acid bacteria strains of the embodiments also have beneficial characteristics for patients having suffered from colorectal cancer and that have been treated for the colorectal cancer. The histamine-producing lactic acid bacterial strains can thereby be used to reduce the risk of relapse of the colorectal cancer.

In an embodiment, the histamine-producing lactic acid bacterial strain is for use in prophylaxis, inhibition, treatment or reducing a risk of relapse of an inflammation-associated colorectal cancer.

The patient is preferably a mammalian patient and more preferably a human patient. In a particular embodiment, the patient is a male human patient. Thus, in this embodiment, the histamine-producing lactic acid bacterial strain is for use in in prophylaxis, inhibition, treatment or reducing a risk of relapse of colorectal cancer in a male patient.

According to the invention, the histamine-producing lactic acid bacterial strain comprises an active histidine operon. In a particular embodiment this histidine operon comprises, such as consists of, a histidine/histamine antiporter (*hdcP*) gene, a histidine decarboxylase pyruvoyl type *A* (*hdcA*) gene and a histidine decarboxylase pyruvoyl type B *(hdcB)* gene. Thus, in an embodiment the histamine-producing lactic acid bacterial strain comprises the *hdcP* gene, the *hdcA* gene and the *hdcB* gene.

According to the invention, the histamine-producing lactic acid bacterial strain is capable of producing a diacylglycerol kinase (DagK). Experimental data as presented herein shows that it may be advantageous to use a histamine-producing and a DagK-producing lactic acid bacterial strain in the prophylaxis of, inhibition of or treatment of colorectal cancer or reduction of a risk of relapse of colorectal cancer.

DagK production has beneficial effects in terms of suppressing H1R downstream signaling to thereby suppress the inflammatory effects that histamine otherwise may cause. Accordingly, production of both histamine and DagK redirects the action from H1R activation to H2R, which in turn promotes the anti-inflammatory effects of histamine.

In an embodiment, the histamine-producing lactic acid bacterial strain is a histamine-producing *Lactobacillus reuteri* strain. In a particular embodiment, the histamine-producing *L. reuteri* strain is *L. reuteri* ATCC PTA-6475. In another particular embodiment, the histamine-producing *L. reuteri* strain is *L. reuteri* ATCC PTA-4659. In a further particular embodiment, the histamine-producing *L. reuteri* strain is *L. reuteri* DSM 32273. In yet another embodiment, the histamine-producing *L. reuteri* strain is a mixture of at least two *L. reuteri* strains capable of producing histamine and optionally additionally capable of producing DagK. For instance, a mixture of *L. reuteri* ATCC PTA-6475 and ATCC PTA-4659, a mixture of *L. reuteri* ATCC PTA-6475 and DSM 32273, a mixture of *L. reuteri* ATCC PTA-4659 and DSM 32273 or a mixture of *L. reuteri* ATCC PTA-6475, ATCC PTA-4659 and DSM 32273 can be used.

In an embodiment, the histamine-producing lactic acid bacterial strain is capable of suppressing production of at least one cancer-associated cytokine selected from a group consisting of chemokine (C-X-C motif) ligand 1 (CXCL1), interleukin 22 (IL-22) and interleukin 6 (IL-6). CXCL1 is also referred to as GRO1 oncogene, GROα, KC, neutrophil-activating protein 3 (NAP-3) and melanoma growth stimulating activity, alpha (MSGA-α) in the art. These three cytokines are all known to be involved in colorectal cancer. Accordingly, suppression of the production of these cytokines will have beneficial effects in terms of suppressing colon cancer growth, progression and metastasis (due to suppression of CXCL1), suppression of gastric cancer cell invasion and colon cancer stemness (due to suppression of IL-22) and improved colon cancer prognosis (due to suppression of IL-6). Suppression of production of these chemokines can be induced in various ways. For instance, transcription of the cytokine genes can be suppressed or reduced by the histamine-producing lactic acid bacterial strains. Alternatively, or additionally, translation of the cytokine mRNA molecules can be suppressed or reduced by the histamine-producing lactic acid bacterial strains. Also, or additionally, post-translational effects could be involved in order to suppress production of these cytokines. Experimental data as presented herein indicates that the histamine-producing lactic acid bacterial strains of the embodiments reduce the protein levels of these cytokines in plasma and reduced the relative gene expressions of these cytokines.

In an embodiment, the histamine-producing lactic acid bacterial strain is capable of reducing abundance of CD11b⁺Gr-1⁺ immature myeloid cells (IMCs) in the spleen. CD11b⁺Gr-1⁺ IMCs is associated with cancer progression in mammals. Accordingly, reducing the abundance of these CD11b⁺Gr-1⁺ IMCs as shown in the experimental data will have beneficial effects with regard to suppressing cancer progression.

A further aspect of the embodiments relates to a method of selecting a lactic acid bacterial strain for use in prophylaxis, inhibition, treatment or reducing a risk of relapse of colorectal cancer. The method comprises screening lactic acid bacteria for presence of an active histidine operon and presence of expression of a gene encoding a DagK. The method also comprises selecting a lactic acid bacterial strain for use in prophylaxis, inhibition, treatment or reducing a risk of relapse of colorectal cancer identified as a lactic acid bacterial strain having an active histidine operon and producing histamine and producing DagK.

Thus, this aspect of the embodiments relates to a method that can be used to select and identify lactic acid bacterial strains that are suitable for use in prophylaxis, inhibition, treatment or reducing a risk of relapse of colorectal cancer. Such selected lactic acid bacterial strains are identified as having an active histidine operon and producing histamine and producing DagK as described herein.

Presence of an active histidine operon can be decided based on detecting the presence of a histidine/histamine antiportergene, a histidine decarboxylase pyruvoyl type A gene and a histidine decarboxylase pyruvoyl type B gene, such as the presence of the *hdcP* gene, the *hdcA* gene and the *hdcB* gene. Alternatively, presence of an active histidine operon can be decided based on detecting production or presence of a histidine/histamine antiporter protein, a histidine decarboxylase pyruvoyl type A protein and a histidine decarboxylase pyruvoyl type B protein, such as the production or presence of the proteins HdcP, HdcA and HdcB.

According to the invention, screening the lactic acid bacteria comprises screening lactic acid bacteria for presence of the active histidine operon and an additional capability of producing a diacylglycerol kinase (DagK). In this embodiment, selecting the lactic acid bacterial strain comprises selecting a lactic acid bacterial strain for use in prophylaxis, inhibition, treatment or reducing a risk of relapse of colorectal cancer identified as a lactic acid bacterial strain having an active histidine operon and producing histamine and producing DagK.

Hence, in an embodiment the lactic acid bacterial strain does not only comprise the active histidine operon to produce histamine but also has the capability to produce DagK. Capability to produce DagK can be assessed either by detecting presence of a gene encoding a diacylglycerol kinase, such as the *dagK* gene, in the lactic acid bacterial strain, either in the genome thereof or in an expression cassette, such as in a plasmid. Alternatively, capability of producing DagK could be determined by detecting presence of the diacylglycerol kinase protein, such as in the cytosol of the bacteria or, if the bacterial strain additionally is capable of secreting DagK, in the culture medium, in which the bacterial strain is cultured.

In an example of the disclosure, the method of selecting a lactic acid bacterial strain for use in prophylaxis, inhibition, treatment or reducing a risk of relapse of colorectal cancer comprises identifying and selecting a lactic acid bacterial strain other than *L. reuteri* ATCC PTA-4659 and ATCC PTA-6475.

An example of the disclosure relates to method of selecting a lactic acid bacterial strain for use in prophylaxis, inhibition, treatment or reducing a risk of relapse of cancer. The method comprises screening lactic acid bacteria for presence of an active histidine operon and a capability of producing a diacylglycerol kinase (DagK). The method also comprises selecting a lactic acid bacterial strain for use in prophylaxis, inhibition, treatment or reducing a risk of relapse of cancer identified as a lactic acid bacterial strain having an active histidine operon and being capable of producing histamine and producing DagK.

In this example of the disclosure, the selected lactic acid bacterial strain does not necessarily have to be used to prevent, inhibit, treat or reduce the risk of relapse of colorectal cancer. In clear contrast, the capability of producing not only histamine but also a diacylglycerol kinase will be beneficial also in other types of cancer in addition to colorectal cancer, including melanoma, breast cancer, pancreas cancer and the like.

In an example of the disclosure, selecting the lactic acid bacterial strain comprises selecting a lactic acid bacterial strain for use in prophylaxis, inhibition, treatment or reducing a risk of relapse of a histamine-associated cancer selected from a group consisting of colorectal cancer, melanoma, breast cancer and pancreas cancer identified as a lactic acid bacterial strain having an active histidine operon and being capable of producing histamine and producing DagK.

In an example of the disclosure, the method of selecting a lactic acid bacterial strain for use in prophylaxis, inhibition, treatment or reducing a risk of relapse of cancer comprises identifying and selecting a lactic acid bacterial strain other than *L. reuteri* ATCC PTA-4659 and ATCC PTA-6475.

Yet another example of the disclosure relates to a method of selecting a lactic acid bacterial strain for use in prophylaxis, inhibition or treatment of an inflammatory condition. The method comprises screening lactic acid bacteria for presence of an active histidine operon and a capability of producing a diacylglycerol kinase (DagK). The method also comprises selecting a lactic acid bacterial strain for use in prophylaxis, inhibition or treatment of an inflammatory condition identified as a lactic acid bacterial strain having an active histidine operon and being capable of producing histamine and producing DagK.

A lactic acid bacterial strain capable of producing both histamine and diacylglycerol kinase will, when administered to a patient, have beneficial effects in terms of preventing, reducing or treating various inflammatory conditions. The produced DagK will suppress the inflammatory properties of histamine by suppressing the H1R downstream signaling or pathway. Accordingly, histamine may exert anti-inflammatory properties due to activation of H2R.

Patients suffering from colorectal cancer show lack of IMC's maturation due to histamine deficiency. Accordingly, it may be beneficial to supplement these patients with histamine at biological levels. However, these patients also show increased pro-inflammatory response and giving them histamine molecule might lead to adverse effects. Therefore, the best therapeutic approach might to provide a beneficial bacterial strain that has the ability to supply exogenous histamine and also suppress inflammatory responses by inhibition the pro-inflammatory signaling. Therefore, a lactic acid bacterial strain capable of producing both histamine and DagK is of huge interest to treat pro-inflammatory disorders with myeloid dysfunction.

**In** an example of the disclosure, selecting the lactic acid bacterial strain comprises selecting a lactic acid bacterial strain, for use in prophylaxis, inhibition or treatment of an inflammatory condition selected from a group consisting of colitis, inflammatory bowel disease, irritable bowel syndrome, diverticulosis, gingivitis, mastitis and vaginitis, identified as a lactic acid bacterial strain having an active histidine operon and being capable of producing histamine and producing DagK.

Thus, the above disclosed inflammatory conditions are illustrative, but preferred examples of inflammatory conditions that can be prevented, inhibited or treated by lactic acid bacterial strains identified and selected as disclosed herein.

In an example of the disclosure, the method of selecting a lactic acid bacterial strain for use in prophylaxis, inhibition or treatment of an inflammatory condition comprises identifying and selecting a lactic acid bacterial strain other than *L. reuteri* ATCC PTA-4659 and ATCC PTA-6475.

Further aspects of the embodiments relates to a *Lactobacillus reuteri* DSM 32273, which is a histamine- and DagK-producing *L. reuteri* strain.. *Lactobacillus reuteri* strain DSM 32273 was deposited under the Budapest Treaty at the DSMZ-Deutsche Sammlung von Mikroorganismen und Zellkulturen GmbH (Inhoffenstrasse 7B, D - 38124 Braunschweig) on March 8, 2016. This new *L. reuteri* DSM 32273 may be used as a medicament. For instance, *L. reuteri* DSM 32273 can be used in prophylaxis, inhibition, treatment or reducing a risk of relapse of colorectal cancer. *L. reuteri* DSM 32273 can also be used in in prophylaxis, inhibition or treatment of an inflammatory condition. In a particular embodiment, the inflammatory condition is selected from a group consisting of colitis, inflammatory bowel disease, irritable bowel syndrome, diverticulosis, gingivitis, mastitis and vaginitis. Further uses of *L. reuteri* DSM 32273 include amelioration of infantile colic, alleviation of eczema, reduction of episodes of workplace illness, suppression of Heliobacter pylori infection.

The embodiments also include use of a histamine-producing lactic acid bacterial strain for the manufacture of a medicament for prophylaxis, inhibition, treatment or reducing a risk of relapse of colorectal cancer, and use of a histamine-producing lactic acid bacterial strain for the manufacture of an adjuvant in a cancer treatment selected from a group consisting of radiotherapy and chemotherapy. In these embodiment, the histamine-producing lactic bacterial strain is also capable of producing and optionally secreting a diacylglycerol kinase.

The disclosure provides a method for prophylaxis, inhibition, treatment or reducing a risk of relapse of colorectal cancer in a subject. The method comprises administering an effective amount of a histamine-producing lactic acid bacterial strain to the patient. The disclosure also provides a method for prophylaxis, inhibition or treatment of a gastrointestinal disturbance associated with a cancer treatment selected from a group consisting of radiotherapy and chemotherapy. The method comprising administering an effective amount of a histamine-producing lactic acid bacterial strain to a patient subject or to be subject to the cancer treatment. The histamine-producing lactic bacterial strain is also capable of producing and optionally secreting a diacylglycerol kinase. The patient is preferably a mammalian patient and more preferably a human patient, such as a male human patient.

An appropriate mode of administration and formulation of the strains is chosen depending on the site where local production of histamine and DagK is desired. A preferred mode of administration is oral, however, equally for some treatments topical or some other form of local administration to the skin, rectum, vagina or gums will be appropriate, or intravenous or intramuscular injection will be appropriate.

Dietary mixtures comprising histidine may be used to ensure the presence of histidine and thereby increase the efficacy of the bacteria. Histidine may be administered alone or together with the bacteria.

One possibility to ensure the bacteria's supply of histidine is to eat histidine rich food, including but not limited to soy protein, cheese, egg, chicken and pork.

### EXAMPLES

### EXAMPLE 1

### Bacterial Strains and Culture Conditions

*L. reuteri* ATCC PTA 6475 (deposited under the Budapest Treaty at the ATCC-American Type Culture Collection (10801 University Boulevard, Manassas, VA 20110 USA) on December 21, 2004) and its *hdcA* mutant as described previously (Thomas et al., 2012) were used to colonize the mice. Both strains were cultured at 37°C in deMan, Rogosa, Sharpe media (Difco, Franklin Lakes, NJ) in an anaerobic workstation (MACS MG-500, Microbiology International, Frederick, MD) supplied with a mixture of 10% CO₂, 10% H₂, and 80% N₂.

### Animals

*Hdc*^{*-*/*-*} BALB/c mice were originally provided by Timothy C. Wang (Columbia University) and rederived at Baylor College of Medicine. Rederived *Hdc*^{*-*/*-*} mice were maintained under specific pathogen-free (SPF) conditions at Texas Children' Hospital. Mice were kept under filter top cages (5 mice per cage) and had free access to distilled water and PicoLab Rodent 50IF/6F diet. All mouse experiments were performed in a SPF animal facility, according to an Institutional Animal Care and Use Committee (IACUC)-approved mouse protocol at Baylor College of Medicine, Houston, TX.

### Preparation of Bacteria and Administration to Mice

*L. reuteri* strains and culture conditions were described above. Bacteria were harvested at exponential phase (5.5 hours in MRS media with an initial OD₆₀₀ₙₘ = 0.03), centrifuged at 2500 × g for 4 min and the bacterial pellet was resuspended in sterile MRS media for animal feeding. All *L. reuteri* strains were prepared freshly before administration to mice. Each mouse received 5×10⁹ CFU of bacteria in 0.2 ml MRS or MRS media only as control by orogastric gavage. The frequency of bacteria administration was once per day for seven days before AOM injection and once per three days afterwards for 15 weeks with a pause when the mice received DSS challenge.

### Induction of Colon Cancer in Balb/c Mice

At 12 weeks of age, mice in the positive control group and bacteria treated groups received a single dose of the genotoxic colonic carcinogen AOM (12.5 mg per kg body weight) by intraperitoneal injection. These mice were challenged with two cycles of 2% (w/v) DSS in drinking water for 6 days, with one cycle immediately after AOM injection, followed by a recovery period with drinking water for two weeks before the second cycle. Mice in the negative control group received one dose of buffered saline solution (PBS) instead of AOM and drinking water.

### Tumor Assessment and Tissue Preparations

Fifteen weeks following AOM injection, mice were sacrificed and specimens were collected as follows. Blood was collected from sedated mice via cardiac puncture in blood sample collection tubes with K₂EDTA (Becton, Dickinson and Company, Franklin Lakes, NJ), centrifuged at 17000 × g for 10 min at 4°C to isolate plasma. The gastrointestinal tract was carefully removed and luminal contents of ileum, cecum and colon were collected and flash frozen in liquid nitrogen. The mouse colons were excised and opened longitudinally, and the number and size of tumors were counted and measured blindly. Intestinal mucosa was scraped with an operating knife blade and stored in RNALater (Ambion, Austin, TX) to analyze the mRNA expression levels in the future. All the samples were stored at -80°C until analyzed. Mouse intestines were fixed in 10% formalin, embedded with paraffin, and microtome-sectioned at 5 µm. The sectioned tissues were used for histology and immunohistochemistry studies targeting H2R expression using specific antibody (Alomone Labs, Jerusalem, Israel and Abcam plc, MA, US). Mouse spleen samples were collected immediately after sacrificing mice. These samples were used for flow cytometry studies.

### Statistical Analysis

Biostatistical analysis was performed using GraphPad Prism (version 5) software (GraphPad Inc., La Jolla, CA). For numeric variables that fit normal distribution (determined using the Kolmogorov-Smirnov test), data were presented as arithmetic means with standard deviations, and different groups were compared with the t test (two groups) or one-way ANOVA (more than two groups). Otherwise, data were presented as box and whiskers plots showing the median values, 10th and 90th percentiles or scatter plots showing the median values. Different groups were compared by a non-parametric Mann-Whitney U test (two groups) or Kruskal-Wallis test. Differences between the groups were considered significant at **P* < *0.05, **P<0.01, ***p<0.001.*

### EXAMPLE 2

### Preparations as described in Example 1.

### Flow Cytometric Analysis

Bone marrow-derived cells from the femurs and tibia of mice from each group were immediately flushed with ice-cold DMEM (ATCC, cat. 30-2002) containing 10% FBS. This procedure was followed by the addition of red blood cell lysis buffer to deplete RBCs (BD Biosciences). Spleens were removed and stored in ice cold DMEM (ATCC, cat. 30-2002) with 10% FBS. This step was followed by the isolation of the spleen cells using sterile glass slides and addition of RBC lysis buffer is added to the isolated cells. Single-cell suspensions were made by filtering the cells through 40-µm filter strains. For flow cytometric analysis, single-cell suspensions were stained with antibodies [1 µl APC-Cy7-conjugated anti-Gr-1 (BD Pharmingen, cat. 557661), and 5 µl FITC-conjugated anti-CD11b (BD Pharmingen, cat. 557396)] for 30 min on ice, in the dark and evaluated by multicolor flow cytometry using a BD FACSCanto cell analyzer and data collected with FACSDiva software (BD Biosciences). The accumulated data were analyzed with FlowJo V10 software (FlowJo, LLC).

### EXAMPLE 3

### Preparations as in Example 1.

### Cytokine Measurement by Multiplex Immunoassay in the Mouse Plasma

The concentrations of murine IFN-γ, IL-1α, IL-1β, IL-4, IL-6, IL-10, IL-12, IL-13, IL-17A, KC, TNF, IL-21, IL-22, and IL-23 in the plasma were measured using cytokine multiplex kits (Millipore, Billerica, MA, USA), see Table 1. Quantification of cytokines was performed using the Luminex system (Austin, TX, USA) according to the manufacturer's instructions. Briefly, 25 µl plasma samples collected above from each mouse were thawed completely and diluted with the same amount of Assay Buffer provided in the kits. The assays were performed in duplicate blindly. The reports automatically generated by MILLIPLEX^{®} Analyst 5.1 Software were reviewed, and only cytokines that were beyond the limit of detection value and below the saturation value were considered.

**Table 1. Cytokines Measured in the Four Multiplex Kits for the Luminex Assay.**

| Catalogue Number | Cytokines measured by the kit |
|---|---|
| MCYTOMAG-70K-12 | IFN-g, IL-1a, IL-1b, IL-4, IL-6, IL-10, IL-12 (P40), IL-12 (P70), IL-13, IL-17A, KC, TNF-a |
| MCYP2MAG-73K-02 | IL-21, IL-22 |
| MCYP3MAG-74K-01 | IL-23 |
| MAGPMAG-24K-01 | EGF |

### EXAMPLE 4

### Preparations as in Example 1.

### mRNA levels of Cytokines and Histamine Receptors in Colonic Mucosa

To quantify the relative mRNA expression levels of interferon (IFN)-y, tumor necrosis factor (TNF), interleukin (IL)-6, IL-12, IL-23, IL-17, IL-18, Il-22, IL-4, KC and histamine H2 receptor (H2R), RNA was extracted from colonic mucosa samples using the miRNeasy^{®} mini kit (Qiagen, Hilden). One µg of RNA was reverse-transcribed to single-stranded cDNA using the RevertAid H minus First Strand cDNA Synthesis Kit (ThermoFisher Scientific, USA). Reverse transcriptase real-time (RT) PCR was performed using Real-Time PCR system (Stratagene). The RT-PCR reaction mix (adjusted with H₂O to a total volume of 25 µl) contained 1 µl template DNA, 12.5 µl Power SYBR Green PCR master mix (ABI, Life Tech), and 0.5 µl of the respective primers (10 µM each). The forward and reverse primers used for IFN-γ, IL-12, IL-17, TNF-α, IL-6, IL-23, IL-18 and IL-4 quantification were described previously (Ganesh et al., 2012) and the primers for other genes were shown in Table 2. Relative mRNA target gene expression levels (Ratio = [(E_{target}) ^{dCPtarget (Control-Sample)}] / [(E_{ref.}) ^{dCPref. (Control-Sample)}]) were normalized to the housekeeping gene, glyceraldehyde 3-phosphate dehydrogenase (GAPDH), and used as a reference. Subsequently, intestinal mucosal cytokine and H2R gene expression values of the control group were set to 1.0 and used as the calibrator to identify the relative mRNA fold difference between the negative control group (MRS/PBS-H2O), positive control group (MRS/AOM-DSS), *L. reuteri* ATCC PTA-6475-treated group *(L. reuteri* 6475/AOM-DSS) and isogenic *L. reuteri hdcA* mutant-treated group *(hdcA* mutant/AOM-DSS).

**Table 2. Primers and Probes Used for the Gene Expression Studies.**

| Mouse gene | Primers (5'-3') | | SEQ ID NO: | |
|---|---|---|---|---|
| | Forward | Reverse | Forward | Reverse |
| IL-1α | cagagagggagtcaactcattg | gtttctggcaactccttcagc | 1 | 2 |
| KC | gactccagccacactccaac | tgacagcgcagctcattg | 3 | 4 |
| IL-22 | tgacgaccagaacatccaga | aatcgccttgatctctccac | 5 | 6 |
| IL-10 | cagagccacatgctcctaga | tgtccagctggtcctttgtt | 7 | 8 |
| SPDEF | cctcctggtccctgaggt | cagtgaatgtggccctgac | 9 | 10 |
| H2R | taagcgacccggtacagc | tcgatggcttaaggtacaacac | 11 | 12 |
| GAPDH | gccaaaagggtcatcatctc | cacacccatcacaaacatgg | 13 | 14 |

### EXAMPLE 5

### Preparations as in Example 1.

### PET Imaging of Living Mice

PET/CT scanning was performed 15 weeks after AOM injection, just before sacrificing the mice as described (Brewer et al., 2008) with minor modifications. Briefly, mice were anesthetized with isoflurane and received 200 µCi ¹⁸F-FDG by intraperitoneal (IP) injection. One hour later, these mice received 200 µL MD-Gastroview rectally via a 3.5F catheter immediately before scan initiation. Computed tomography (CT) scan was performed for 10 min followed by a PET scan for 20 min using the Inveon PET/CT Multimodality System (Siemens, Germany). Mice were kept sedated during the scanning process by constant isoflurane inhalation. The images were recorded and FDG standardized uptake values (SUVs) were analyzed blindly using Inveon Research Workplace software (Siemens, Germany). The 2D images of mice were generated by OsiriX Imaging software (Pixmeo, Swiss).

### EXAMPLE 6

### Quantification of dagK mRNA Gene Expression by qRT-PCR

Wild-type *Lactobacillus reuteri* ATCC PTA-6475, *hdcA* mutant *L. reuteri* 6475, wild-type *L. reuteri* ATCC PTA-4659 (deposited under the Budapest Treaty at the ATCC-American Type Culture Collection (10801 University Boulevard, Manassas, VA 20110 USA) on September 11, 2002) and wild-type *L. reuteri* DSM 17938 (deposited under the Budapest Treaty at the DSMZ-Deutsche Sammlung von Mikroorganismen und Zellkulturen GmbH (Mascheroeder Weg 1b, D-38124 Braunschweig) on January 30, 2006) were grown in the MRS media overnight at 37°C and were cultured under strict anoxic conditions N₂ / CO₂ (80/20; v/v) as the gas phase. 100 µl of fresh culture was incubated into 10 ml of lactobacillus defined media 4 (LDM4). The cultures were maintained in mini bioreactors at 37°C under strict anoxic conditions. The samples were collected at 3 hours, 6 hours, 24 hours and 48 hours. The bacterial pellet was obtained by treating the culture at 6000 × g for 10 min at 4°C. The pellet was treated with RNase. mRNA from the bacterial cells were extracted by Trizol separation kit. 500 ng of mRNA from each group was used to convert mRNA into cDNA. The treated cDNA is diluted 1:2 and was used to run qRT-PCR. The Stratagene Mx3000p (Agilent Technologies GmbH, USA) qRT-PCR was used for amplification and fluorescent data collection. The master mix consisted of 12.5 µl Power SYBR Green 2000 (ABI systems, USA), 0.5 µl of each primer (10 µM), 1 µl of sample and adjusted with water to a final volume of 25 µl per well. After PCR amplification, the specificity of the primers was checked by inspecting the melting curve and determining the size of the amplicon by agarose gel electrophoresis (1 %). Relative mRNA target gene expression levels (Ratio = [(E_{target}) ^{dCPtarget(control-sample)}] / [(E_{ref.}) ^{dCPref. (control-sample)}]) were normalized to the house keeping gene *rpoB* and used as a reference. Subsequently, mRNA obtained from 3 hours culture of each bacterium were set to 1.0 and used as the calibrator to identify the relative mRNA fold difference of same bacterial strain at different time points like 6 hrs, 24 hrs and 48 hrs of *L. reuteri* ATCC PTA-6475, ATCC PTA-4659 and DSM 17938.

Figure 9 illustrates the results of the *dagK* gene expression experiments. The figure shows an increased *dagK* expression by both wild-type and *hdcA* mutant *L. reuteri* ATCC PTA-6475 together with *L*. *reuteri* ATCC PTA-4659. However, *L. reuteri* DSM 17938 which cannot produce histamine also lacked *dagK* expression. Interestingly *dagK* mRNA expression was expressed very high during the elongation phase of the bacteria. From the repetition experiments 12 hrs incubation time points was selected since it showed similar expression like 6 hrs.

### EXAMPLE 7

### LC-MS/MS for Detecting DagK Protein in the Bacterial Culture Supernatants

According to the literatures DagK is believed to have soluble isoforms in gram positive bacteria. We hypothesized that DagK is released from *L. reuteri* ATCC PTA-6475 and it interacts with the host intestinal epithelial lipid signaling and promotes anti-inflammatory behavior under inflammatory circumstances together with histamine release. When we mutated the *dagK* gene in *L. reuteri* ATCC PTA-6475 and colonized our germ-free (GF) mice with the DAGK mutant *L. reuteri* ATCC PTA-6475 we did not see a suppression of IL-6 and IL-1α like we observed in the germ-free mice colonized with wild-type *L. reuteri* ATCC PTA-6475. The basal pro-inflammatory cytokine levels were significantly suppressed. This brought us to a conclusion that *L. reuteri* ATCC PTA-6475 needs histamine for H1R and H2R activation. However, H1R downstream signaling is interrupted by DagK synthesis in *L. reuteri* by inhibiting lipid DAG involved in the signaling and thereby suppress pro-inflammatory effect of histamine. This allows only H2R activation by *L. reuteri* derived histamine and H2R activation is known to promote anti-inflammatory symptoms.

For dagK to show any positive effect on host immune response host-DAG lipid should be expressed. For DAG to be activated H1R signaling must be activated. That is why when we mutated the *dagK* in *L. reuteri* and colonized the mice we did not see pro-inflammatory cytokine suppression. This was additionally confirmed with PKC and PKA activation. In addition, we did not see any difference in H1R and H2R expression on the tissue (f-IHC) between the groups colonized with *L. reuteri* wild-type or *hdcA* mutant or *dagK* mutant strains because these germ-free mice also expressed endogenous histamine.

When HDC-knock out mice received *hdcA* mutant *L. reuteri,* they could not protect themselves from inflammation and cancer because they lacked both endogenous and exogenous histamine. But in GF mice colonized with *hdcA* mutant *L. reuteri,* the endogenous histamine was present, which activated the receptor. However, DagK produced by the *hdcA* mutant *L. reuteri* helped suppress the expression of H1R activation. That is why we saw suppressed pro-inflammatory biomarkers in *hdcA* mutant colonized groups. But when *dagK* is knocked out in *L. reuteri (dagK* mutant) there was endogenous and exogenous histamine activating both H1R and H2R but no DagK to suppress the H1R. Accordingly, we saw increased pro-inflammatory signalling similar to GF mice without any bacteria. In GF mice the endogenous histamine activates both H1R and H2R.

Experimental data thereby showed that the basal immune levels were shut down after colonizing the mice with wild-type *L. reuteri* ATCC PTA-6475, which thereby is a complete immune-suppressor and in patients with aggressive immune response this bacterial strain can be a great therapeutics.

To further show whether DagK isoforms are secreted or not from *L. reuteri* we performed bacterial cell culture experiment. 100 µl of overnight MRS grown *L. reuteri* ATCC PTA-6475 at 37°C under anoxic conditions were added to 10 ml LDM4 media and left at 37°C for 12 hours under anoxic conditions. The bacterial cells were removed by centrifugation at 6000 × g, 10 min at 4°C. 1:1 ratio of proteinase and protein kinase inhibitor was added to the supernatant. The supernatants were filtered by 0.22 µm filter to remove traces of bacteria. Since DagK is a 10-13 kDa protein, it is necessary to reduce the background. Therefore the supernatant was processed with 50 kDa filtrate. The flow through was added to the 3 kDa filtrate and spin at 5000 × g for 30 min. The concentrate on the upper phase was used to run the LC-MS/MS after tryptic digestion. Figure 10 illustrates the amino acid sequence of DagK protein form *L. reuteri* ATCC PTA-6475 together with trypsin digestion or cleavage sites (Tryps).

The results of the LC-MS/MS experiment is presented in Table 3 and Figure 10. The sequences that match the *L. reuteri* DagK protein were found in the supernatant. Accordingly, *L. reuteri* ATCC PTA-6475 is capable of producing and secreting the DagK protein.

**Table 3. LC-MS/MS Results of Trypsin Treatment of Supernatant from L. reuteri ATCC PTA-6475**

| Peptide | -10lgP | Mass | Length | ppm | m/z | RT | Scan | #Spec |
|---|---|---|---|---|---|---|---|---|
| A | 18.31 | 833.3813 | 6 | -6.4 | 417.6953 | 58.33 | 139 | 3 |
| B | 14.72 | 971.5287 | 11 | -62.2 | 486.7414 | 63.33 | 432 | 1 |
| C | 9.09 | 884.4352 | 7 | 35 | 443.2404 | 72.68 | 1088 | 1 |
| D | 8.83 | 1487.774 | 13 | -64.8 | 744.8461 | 52.15 | 42 | 1 |
| E | 7.94 | 996.4447 | 7 | 30.9 | 499.245 | 64.98 | 549 | 1 |
| F | 5.73 | 2710.64 | 27 | -79.9 | 678.6131 | 86.5 | 1394 | 1 |

### Peptides A-F in Table 3:

| | | |
|---|---|---|
| A | EERNMR | SEQ ID NO: 15 |
| B | DVAAGGVLISA | SEQ ID NO: 16 |
| C | DKHQTEK | SEQ ID NO: 17 |
| D | NMRYHLLAACLAI | SEQ ID NO: 18 |
| E | EERNMRY | SEQ ID NO: 19 |
| F | KAKDVAAGGVLISAIFSVLVGLIIFIP | SEQ ID NO: 20 |

### EXAMPLE 8

### Identification of Strains Capable of Producing DagK

The bacteria are cultivated on MRS plates for 16 h at 37°C in anaerobic atmosphere. Bacterial colonies are collected with a sterile plastic loop and suspended in 100 µl of sterile water (PCR quality). Alternatively, DNA can be prepared from the bacterial culture using any suitable method, see for instance Example 6.

Presence of the *dagK* gene is examined by PCR, e.g. by using PuReTaq Ready To Go PCR beads (GE HealthCare) and the primer pair dagK_LrF (TGGACTCACGCGATAAACATCA, SEQ ID NO: 21) and dagK_LrR (ACAATCAAATCTGTAACAGCTTCG, SEQ ID NO: 22), 0.4 mM of each. Bacterial suspension or DNA preparation (0.5 µl) is added to the PCR mix and the PCR reaction is performed by running the program 95°C, 5 min; 30× (95°C, 30 s; 58°C, 30 s; 72°C, 30 s); 72°, 10 min. The PCR products are separated and visualized by using standard agarose gel electrophoresis and the sequence is determined by standard Sanger sequencing using the forward primer (dagK _LrF) used for the PCR.

### EXAMPLE 9

### Analysis of Lactobacillus reuteri DSM 32273 Capable of Producing Histamine and DagK

*Lactobacillus reuteri* DSM 32273 (deposited under the Budapest Treaty at the DSMZ-Deutsche Sammlung von Mikroorganismen und Zellkulturen GmbH (Inhoffenstrasse 7B, D - 38124 Braunschweig) on March 8, 2016) bacteria were grown over night in MRS broth at 37°C. The bacterial suspensions was centrifuged at 3500 rpm for 5 min and 1 µl of the pellet was suspended in 100 µl of PBS.

PCR analysis (histidine decarboxylase (*hdc*)) was performed with the following primers: hdcA425fde (CGTCAYTATCCWGCTCCWGG, SEQ ID NO: 23) and hdcA867rde (TCCATRTCAGTATCWGGKGT, SEQ ID NO: 24). The resulting PCR product had a size of 442 bp. The PCR primers were designed from an alignment of *hdc* from *L. reuteri, L. hilgardii, L. buchneri* and *L. sakei.* DreamTaq Green PCR Mastermix (2X Thermo Scientific, article number K1081) was used for the PCR reactions. PCR reactions according to this:

| Mastermix with primers | Volume per reaction |
|---|---|
| Water (PCR quality) | 10.0 µl |
| Primer, forw. (10 pmol/µl) | 1.0 µl |
| Primer, rev. (10 pmol/µl) | 1.0 µl |
| DreamTaq | 12.5 µl |

24.5 µl of the mastermix was mixed with 0.5 µl bacterial suspension and the following PCR program was run: 95°C, 10 min; 30× (95°C, 30 s; 48°C, 30 s; 72°C, 30 s); 72°C, 5 min.

The PCR analysis of *dagK* gene in *L. reuteri* DSM 32273 was done as described in Example 8.

The results showed that *L. reuteri* DSM 32273 was positive for the gene encoding histidine decarboxylase and the *dagK* gene, see Table 4. The bacterial strains *L. reuteri* ATCC PTA-6475 and DSM 17938 were included as controls.

**Table 4. Results from the PCR Analysis Showing the Species, Strain and Host Origin of the Tested bacteria.**

| Species | Strain | Host origin | Presence of *hdc* gene | Presence of *dagK* gene |
|---|---|---|---|---|
| *L. reuteri* | ATCC PTA-6475 | Human | + | + |
| | DSM 17938 | Human | - | - |
| | DSM 32273 | Human | + | + |

The embodiments described above are to be understood as a few illustrative examples of the present invention. It will be understood by those skilled in the art that various modifications, combinations and changes may be made to the embodiments without departing from the scope of the present invention. In particular, different part solutions in the different embodiments can be combined in other configurations, where technically possible. The scope of the present invention is, however, defined by the appended claims.

### REFERENCES

Adams, W.J., and Morris, D.L. (1994). Short-course cimetidine and survival with colorectal cancer. Lancet 344, 1768-1769.
Brewer, S., McPherson, M., Fujiwara, D., Turovskaya, O., Ziring, D., Chen, L., Takedatsu, H., Targan, S.R., Wei, B., and Braun, J. (2008). Molecular imaging of murine intestinal inflammation with 2-deoxy-2-[18F]fluoro-D-glucose and positron emission tomography. Gastroenterology 135, 744-755.
Frei, R., Ferstl, R., Konieczna, P., Ziegler, M., Simon, T., Rugeles, T.M., Mailand, S., Watanabe, T., Lauener, R., Akdis, C.A., et al. (2013). Histamine receptor 2 modifies dendritic cell responses to microbial ligands. The Journal of allergy and clinical immunology 132, 194-204.
Fukui, H., Zhang, X., Sun, C., Hara, K., Kikuchi, S., Yamasaki, T., Kondo, T., Tomita, T., Oshima, T., Watari, J., et al. (2014). IL-22 produced by cancer-associated fibroblasts promotes gastric cancer cell invasion via STAT3 and ERK signaling. British journal of cancer 111, 763-771.
Galitovskiy, V., Kuruvilla, S., Sevriokov, E., Corches, A., Pan, M., Kalantari-Dehaghi, M., Chernyavsky, A., Mukherjee, J., and Grando, S. (2013). Development of novel approach to diagnostic imaging of lung cancer with 18F-Nifene PET/CT using A/J Mice treated with NNK. J Cancer Res Ther 1, 128-137.
Ganesh, B.P., Richter, J.F., Blaut, M., and Loh, G. (2012). Enterococcus faecium NCIMB 10415 does not protect interleukin-10 knock-out mice from chronic gut inflammation. Beneficial microbes 3, 43-50.
Garcia-Caballero, M., Neugebauer, E., Campos, R., Nunez de Castro, I., and Vara-Thorbeck, C. (1988). Increased histidine decarboxylase (HDC) activity in human colorectal cancer: results of a study on ten patients. Agents Actions 23, 357-360.
Ji, Y., Yang, X., Li, J., Lu, Z., Li, X., Yu, J., and Li, N. (2014). IL-22 promotes the migration and invasion of gastric cancer cells via IL-22R1/AKT/MMP-9 signaling. International journal of clinical and experimental pathology 7, 3694-3703.
Jutel, M., Watanabe, T., Klunker, S., Akdis, M., Thomet, O.A., Malolepszy, J., Zak-Nejmark, T., Koga, R., Kobayashi, T., Blaser, K., et al. (2001). Histamine regulates T-cell and antibody responses by differential expression of H1 and H2 receptors. Nature 413, 420-425.
Kelly, M.D., King, J., Cherian, M., Dwerryhouse, S.J., Finlay, I.G., Adams, W.J., King, D.W., Lubowski, D.Z., and Morris, D.L. (1999). Randomized trial of preoperative cimetidine in patients with colorectal carcinoma with quantitative assessment of tumor-associated lymphocytes. Cancer 85, 1658-1663.
Kryczek, I., Lin, Y., Nagarsheth, N., Peng, D., Zhao, L., Zhao, E., Vatan, L., Szeliga, W., Dou, Y., Owens, S., et al. (2014). IL-22(+)CD4(+) T cells promote colorectal cancer stemness via STAT3 transcription factor activation and induction of the methyltransferase DOT1L. Immunity 40, 772-784.
Landskron, G., De la Fuente, M., Thuwajit, P., Thuwajit, C., and Hermoso, M.A. (2014). Chronic inflammation and cytokines in the tumor microenvironment. Journal of immunology research 2014, 149185.
Lee, Y.S., Choi, I., Ning, Y., Kim, N.Y., Khatchadourian, V., Yang, D., Chung, H.K., Choi, D., LaBonte, M.J., Ladner, R.D., et al. (2012). Interleukin-8 and its receptor CXCR2 in the tumour microenvironment promote colon cancer growth, progression and metastasis. British journal of cancer 106, 1833-1841.
Nagasaki, T., Hara, M., Nakanishi, H., Takahashi, H., Sato, M., and Takeyama, H. (2014). Interleukin-6 released by colon cancer-associated fibroblasts is critical for tumour angiogenesis: anti-interleukin-6 receptor antibody suppressed angiogenesis and inhibited tumour-stroma interaction. British journal of cancer 110, 469-478.
O'Mahony, L., Akdis, M., and Akdis, C.A. (2011). Regulation of the immune response and inflammation by histamine and histamine receptors. The Journal of allergy and clinical immunology 128, 1153-1162.
Oquendo, P., Alberta, J., Wen, D.Z., Graycar, J.L., Derynck, R., and Stiles, C.D. (1989). The platelet-derived growth factor-inducible KC gene encodes a secretory protein related to platelet alpha-granule proteins. The Journal of biological chemistry 264, 4133-4137.
Thomas, C.M., Hong, T., van Pijkeren, J.P., Hemarajata, P., Trinh, D.V., Hu, W., Britton, R.A., Kalkum, M., and Versalovic, J. (2012). Histamine derived from probiotic Lactobacillus reuteri suppresses TNF via modulation of PKA and ERK signaling. PLoS One 7, e31951.
Tjalsma, H., Boleij, A., Marchesi, J.R., and Dutilh, B.E. (2012). A bacterial driver-passenger model for colorectal cancer: beyond the usual suspects. Nature reviews Microbiology 10, 575-582.
Waldner, M.J., Foersch, S., and Neurath, M.F. (2012). Interleukin-6--a key regulator of colorectal cancer development. International journal of biological sciences 8, 1248-1253.
Watanabe, S., Deguchi, K., Zheng, R., Tamai, H., Wang, L.X., Cohen, P.A., and Shu, S. (2008). Tumor-induced CD11b+Gr-1+ myeloid cells suppress T cell sensitization in tumor-draining lymph nodes. Journal of immunology 181, 3291-3300.
Yang, X.D., Ai, W., Asfaha, S., Bhagat, G., Friedman, R.A., Jin, G., Park, H., Shykind, B., Diacovo, T.G., Falus, A., et al. (2011). Histamine deficiency promotes inflammation-associated carcinogenesis through reduced myeloid maturation and accumulation of CD11b+Ly6G+ immature myeloid cells. Nature medicine 17, 87-95.

## Claims

1. A method of selecting a lactic acid bacterial strain for use in prophylaxis, inhibition, treatment or reducing a risk of relapse of cancer, said method comprising:
screening lactic acid bacteria for presence of an active histidine operon and presence of expression of a gene encoding a diacylglycerol kinase (DagK); and
selecting a lactic acid bacterial strain for use in prophylaxis, inhibition, treatment or reducing a risk of relapse of colorectal cancer identified as a lactic acid bacterial strain having an active histidine operon and producing histamine and producing DagK.

2. A histamine-producing lactic acid bacterial strain for use in prophylaxis, inhibition, treatment or reducing a risk of relapse of colorectal cancer, wherein said histamine-producing lactic acid bacterial strain comprises an active histidine operon and said histamine-producing lactic acid bacterial strain expresses a gene encoding a diacylglycerol kinase (DagK) and produces histamine and produces DagK.

3. The histamine-producing lactic acid bacterial strain for use according to claim 2, wherein said histamine-producing lactic acid bacterial strain is for use in prophylaxis, inhibition, treatment or reducing a risk of relapse of an inflammation-associated colorectal cancer.

4. The histamine-producing lactic acid bacterial strain for use according to claim 2 or 3, wherein said histamine-producing lactic acid bacterial strain is for use in reducing a number of and/or a size of tumors in a patient suffering from colorectal cancer.

5. A histamine-producing lactic acid bacterial strain for use as adjuvant in a cancer treatment of colorectal cancer, wherein said cancer treatment is selected from a group consisting of radiotherapy and chemotherapy, wherein said histamine-producing lactic acid bacterial strain comprises an active histidine operon and expresses a gene encoding a diacylglycerol kinase (DagK) and said histamine-producing lactic acid bacterial strain produces histamine and produces DagK.

6. The histamine-producing lactic acid bacterial strain for use according to any of the claims 2 to 5, wherein said histamine-producing lactic acid bacterial strain is a histamine-producing *Lactobacillus reuteri* strain.

7. The histamine-producing lactic acid bacterial strain for use according to claim 6, wherein said histamine-producing *Lactobacillus reuteri* strain is selected from the group consisting of *Lactobacillus reuteri* ATCC PTA-6475, *Lactobacillus reuteri* ATCC PTA-4659 and *Lactobacillus reuteri* DSM 32273.

8. A *Lactobacillus reuteri* DSM 32273.

9. A *Lactobacillus reuteri* DSM 32273 for use as a medicament.

10. A *Lactobacillus reuteri* DSM 32273 for use in prophylaxis, inhibition, treatment or reducing a risk of relapse of colorectal cancer.

## Patentansprüche

1. Verfahren zum Auswählen eines Milchsäurebakterienstammes zur Verwendung bei der Prophylaxe, Hemmung, Behandlung oder Verringerung des Risikos eines Rückfalls von Karzinomen, wobei das Verfahren Folgendes umfasst:
Screenen von Milchsäurebakterien auf das Vorhandensein eines aktiven Histidin-Operons und das Vorhandensein der Expression eines Gens, das für eine Diacylglycerin-Kinase (DagK) codiert; und
Auswählen eines Milchsäurebakterienstammes zur Verwendung bei der Prophylaxe, Hemmung, Behandlung oder Verringerung des Risikos eines Rückfalls von Kolorektalkarzinom, der als Milchsäurebakterienstamm mit einem aktiven Histidin-Operon identifiziert wurde und Histamin produziert und DagK produziert.

2. Histamin-produzierender Milchsäurebakterienstamm zur Verwendung bei der Prophylaxe, Hemmung, Behandlung oder Verringerung des Risikos eines Rückfalls von Kolorektalkarzinom, wobei der Histamin-produzierende Milchsäurebakterienstamm ein aktives Histidin-Operon umfasst und der Histamin-produzierende Milchsäurebakterienstamm ein Gen exprimiert, das für eine Diacylglycerin-Kinase (DagK) codiert und Histamin produziert und DagK produziert.

3. Histamin-produzierender Milchsäurebakterienstamm zur Verwendung nach Anspruch 2, wobei der Histamin-produzierende Milchsäurebakterienstamm zur Verwendung bei der Prophylaxe, Hemmung, Behandlung oder Verringerung des Risikos eines Rückfalls eines entzündungsassoziierten Kolorektalkarzinoms bestimmt ist.

4. Histamin-produzierender Milchsäurebakterienstamm zur Verwendung nach Anspruch 2 oder 3, wobei der Histamin-produzierende Milchsäurebakterienstamm zur Verwendung bei der Verringerung der Anzahl und/oder der Größe von Tumoren bei einem Patienten, der an Kolorektalkarzinom leidet, bestimmt ist.

5. Histamin-produzierender Milchsäurebakterienstamm zur Verwendung als Adjuvans bei einer Karzinombehandlung von Kolorektalkarzinom, wobei die Karzinombehandlung aus einer Gruppe ausgewählt ist, die aus Strahlentherapie und Chemotherapie besteht, wobei der Histamin-produzierende Milchsäurebakterienstamm ein aktives Histidin-Operon umfasst und ein Gen exprimiert, das für eine Diacylglycerin-Kinase (DagK) codiert, und der Histamin-produzierende Milchsäurebakterienstamm Histamin produziert und DagK produziert.

6. Histamin-produzierender Milchsäurebakterienstamm zur Verwendung nach einem der Ansprüche 2 bis 5, wobei der Histamin-produzierende Milchsäurebakterienstamm ein histaminproduzierender *Lactobacillus-reuteri-Stamm* ist.

7. Histamin-produzierender Milchsäurebakterienstamm zur Verwendung nach Anspruch 6, wobei der Histamin-produzierende *Lactobacillus-reuteri-Stamm* ausgewählt ist aus der Gruppe bestehend aus *Lactobacillus reuteri* ATCC PTA-6475, *Lactobacillus reuteri* ATCC PTA-4659 und *Lactobacillus reuteri* DSM 32273.

8. Ein *Lactobacillus reuteri* DSM 32273.

9. Ein *Lactobacillus reuteri* DSM 32273 zur Verwendung als Arzneimittel.

10. Ein *Lactobacillus reuteri* DSM 32273 zur Verwendung bei der Prophylaxe, Hemmung, Behandlung oder Verringerung des Risikos eines Rückfalls von Kolorektalkarzinom.

## Revendications

1. Procédé de sélection d'une souche bactérienne lactique à utiliser dans la prophylaxie, l'inhibition, le traitement ou la réduction d'un risque de rechute du cancer, ledit procédé comprenant :
le dépistage des bactéries lactiques pour la présence d'un opéron histidine actif et la présence d'expression d'un gène codant pour une diacylglycérol kinase (DagK) ; et
la sélection d'une souche bactérienne lactique à utiliser dans la prophylaxie, l'inhibition, le traitement ou la réduction d'un risque de rechute du cancer colorectal identifié comme une souche bactérienne lactique ayant un opéron histidine actif et produisant l'histamine et produisant DagK.

2. Souche bactérienne d'acide lactique productrice d'histamine à utiliser dans la prophylaxie, l'inhibition, le traitement ou la réduction d'un risque de rechute du cancer colorectal, dans laquelle ladite souche bactérienne d'acide lactique productrice d'histamine comprend un opéron histidine actif et ladite souche bactérienne d'acide lactique productrice d'histamine exprime un gène codant pour une diacylglycérol kinase (DagK) et produit de l'histamine et produit DagK.

3. Souche bactérienne d'acide lactique productrice d'histamine à utiliser selon la revendication 2, ladite souche bactérienne d'acide lactique productrice d'histamine étant destinée à être utilisée dans la prophylaxie, l'inhibition, le traitement ou la réduction d'un risque de rechute d'un cancer colorectal associé à une inflammation.

4. Souche bactérienne d'acide lactique productrice d'histamine à utiliser selon la revendication 2 ou 3, ladite souche bactérienne d'acide lactique productrice d'histamine étant destinée à être utilisée pour réduire le nombre et/ou la taille des tumeurs chez un patient souffrant d'un cancer colorectal.

5. Souche bactérienne d'acide lactique productrice d'histamine à utiliser comme adjuvant dans un traitement du cancer colorectal, ledit traitement du cancer étant choisi dans un groupe constitué de la radiothérapie et de la chimiothérapie, ladite souche bactérienne d'acide lactique productrice d'histamine comprenant un opéron histidine actif et exprimant un gène codant pour une diacylglycérol kinase (DagK) et ladite souche bactérienne d'acide lactique productrice d'histamine produisant l'histamine et produisant DagK.

6. Souche bactérienne d'acide lactique productrice d'histamine à utiliser selon l'une quelconque des revendications 2 à 5, dans laquelle ladite souche bactérienne d'acide lactique productrice d'histamine est une souche de *Lactobacillus reuteri* productrice d'histamine.

7. Souche bactérienne d'acide lactique productrice d'histamine à utiliser selon la revendication 6, dans laquelle ladite souche de *Lactobacillus reuteri* productrice d'histamine est choisie dans le groupe constitué de *Lactobacillus reuteri* ATCC PTA-6475, *Lactobacillus reuteri* ATCC PTA-4659 et *Lactobacillus reuteri* DSM 32273.

8. *Lactobacillus reuteri* DSM 32273.

9. *Lactobacillus reuteri* DSM 32273 à utiliser comme médicament.

10. *Lactobacillus reuteri* DSM 32273 à utiliser pour la prophylaxie, l'inhibition, le traitement ou la réduction du risque de rechute du cancer colorectal.
